(19) Europäisches Patentamt
European Patent Office
Office européen des brevets

(11) Veröffentlichungsnummer: **0 334 055**
**A2**

# (12) EUROPÄISCHE PATENTANMELDUNG

(21) Anmeldenummer: **89103461.3**

(22) Anmeldetag: **28.02.89**

(51) Int. Cl.⁴: **C07D 413/04 , C07D 413/14 , C07D 265/36 , C07D 487/04 , C07D 471/04 , C07D 231/12 , C07D 231/16 , C07D 231/54 , C07D 231/56 , C07D 271/06 , C07D 249/12**

(30) Priorität: **10.03.88 DE 3807836**
**23.09.88 DE 3832348**

(43) Veröffentlichungstag der Anmeldung:
**27.09.89 Patentblatt 89/39**

(84) Benannte Vertragsstaaten:
**BE CH DE FR GB IT LI NL**

(71) Anmelder: **BAYER AG**

**D-5090 Leverkusen 1 Bayerwerk(DE)**

(72) Erfinder: **Schallner, Otto, Dr.**
**Noldeweg 22**
**D-4019 Monheim(DE)**
Erfinder: **Ooms, Pieter, Dr.**
**Doerperhofstrasse 16**
**D-4150 Krefeld 1(DE)**
Erfinder: **Fischer, Reiner, Dr.**
**Rembrandtstrasse 15**
**D-4019 Monheim(DE)**
Erfinder: **Jensen-Korte, Uta, Dr.**
**Geibelstrasse 9**
**D-4000 Düsseldorf 1(DE)**
Erfinder: **Santel, Hans-Joachim, Dr.**
**Grünstrasse 9 a**
**D-5090 Leverkusen 1(DE)**
Erfinder: **Schmidt, Robert R., Dr.**
**Im Waldwinkel 110**
**D-5060 Bergisch-Gladbach 2(DE)**
Erfinder: **Strang, Harry, Dr.**
**Unterdorfstrasse 6 a**
**D-4000 Düsseldorf 31(DE)**

(54) **Substituierte Benzoxazinone, mehrere Verfahren sowie neue Zwischenprodukte zu ihrer Herstellung sowie ihre herbizide Verwendung.**

(57) Die vorliegende Erfindung betrifft substituierte Benzoxazinone der allgemeinen Formel (I)

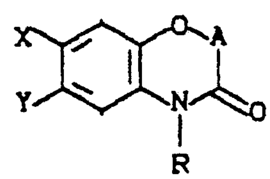

(I)

in welcher
A für Methylen, Ethyliden oder Isopropyliden steht,
R für Wasserstoff oder für einen gegebenenfalls substituierten Rest aus der Reihe Alkyl, Cycloalkyl, Cycloalkylalkyl, Alkenyl, Alkinyl, Aralkyl und Heteroarylalkyl steht,
X für Wasserstoff oder Halogen steht und
Y für die nachstehenden heterocyclischen Gruppierungen steht

EP 0 334 055 A2

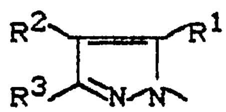 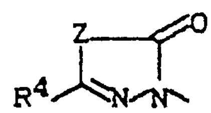

mehrere Verfahren sowie neue Zwischenprodukte zu deren Herstellung und ihre Verwendung als Herbizide.

# Substituierte Benzoxazinone, mehrere Verfahren sowie neue Zwischenprodukte zu ihrer Herstellung sowie ihre herbizide Verwendung

Die vorliegende Erfindung betrifft neue substituierte Benzoxazinone, Verfahren zu ihrer Herstellung sowie ihre Verwendung als Herbizide.

Es ist bekannt, daß bestimmte substituierte Oxadiazolone, wie z. B. 5-tert-Butyl-3-(2,4-dichlor-5-isopropoxy-phenyl)-1,3,4-oxadiazol-2-on (Oxadiazone/Ronstar), herbizide Eigenschaften aufweisen (vgl. US-P 3 835 862). Die Wirkung dieser Verbindungen ist jedoch bei niedrigen Aufwandmengen bzw. Wirkstoffkonzentrationen unbefriedigend.

Es wurden nun die neuen substituierten Benzoxazinone der allgemeinen Formel (I)

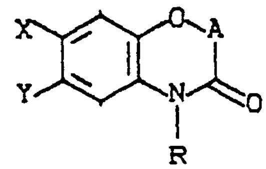

(I)

in welcher

A

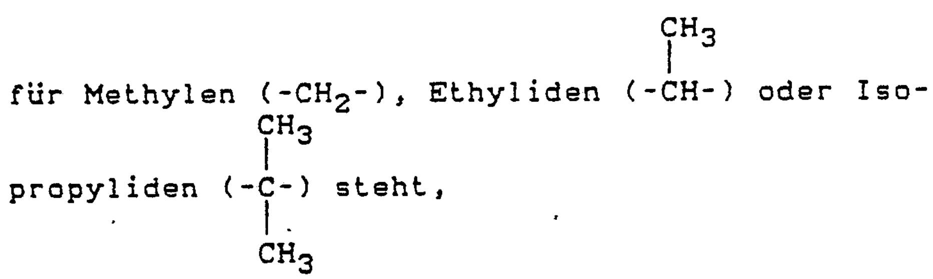


R für Wasserstoff oder für einen gegebenenfalls substituierten Rest aus der Reihe Alkyl, Cycloalkyl, Cycloalkylalkyl, Alkenyl, Alkinyl, Aralkyl und Heteroarylalkyl steht,

X für Wasserstoff oder Halogen steht und

Y für die nachstehenden heterocyclischen Gruppierungen steht

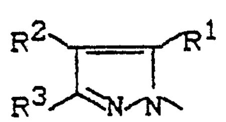

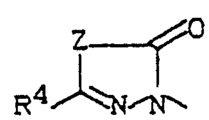

worin

$R^1$ für Wasserstoff, Halogen oder gegebenenfalls durch Halogen substituiertes Alkyl steht,

$R^2$ für Wasserstoff, Cyano, Nitro, Halogen oder gegebenenfalls durch Halogen substituiertes Alkyl steht,

$R^3$ für Wasserstoff oder gegebenenfalls durch Halogen substituiertes Alkyl steht, oder zusammen mit $R^2$ für Alkandiyl steht,

$R^4$ für Wasserstoff oder für jeweils gegebenenfalls durch Halogen substituiertes Alkyl, Cycloalkyl, Alkenyl oder Alkinyl steht und

Z für Sauerstoff oder die Gruppierung $-N-R^5$ steht, worin

$R^5$ für Wasserstoff oder für jeweils gegebenenfalls durch Halogen substituiertes Alkyl, Alkenyl oder Alkinyl steht, oder zusammen mit $R^4$ für Alkandiyl steht,

gefunden.

Weiter wurde gefunden, daß man die neuen substituierten Benzoxazinone der allgemeinen Formel (I) erhält, wenn man

(a) Hydrazino-benzoxazinone der allgemeinen Formel (II)

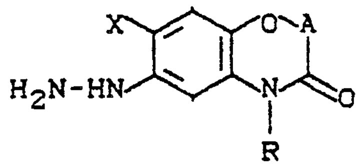

(II)

in welcher
A, R und X die oben angegebenen Bedeutungen haben,
mit 1,3-Dicarbonylverbindungen der allgemeinen Formel (III)

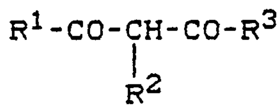

(III)

in welcher
$R^1$, $R^2$ und $R^3$ die oben angegebenen Bedeutungen haben,
bzw. mit Dimethyl- oder Diethyl-Acetalen bzw. -Ketalen von Verbindungen der Formel (III) gegebenenfalls in Gegenwart von Verdünnungsmitteln umsetzt, oder wenn man

(b) die bei Verfahren (a) beschriebenen Hydrazino-benzoxazinone der allgemeinen Formel (II)

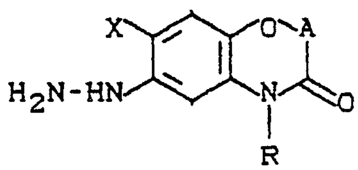

(II)

in welcher
A, R und X die oben angegebenen Bedeutungen haben,
mit N-Alkoxycarbonylcarbonimidsäureestern der allgemeinen Formel (IV)

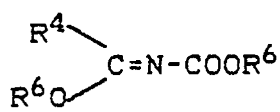

(IV)

in welcher
$R^4$ die oben angegebene Bedeutung hat und
$R^5$ für Niederalkyl steht,
gegebenenfalls in Gegenwart eines Verdünnungsmittels umsetzt, oder wenn man

(c) Acylhydrazino-benzoxazinone der allgemeinen Formel (V)

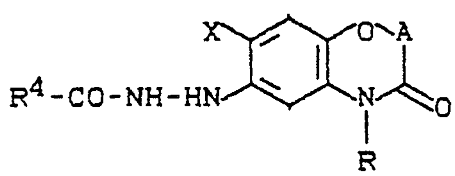

(V)

in welcher
A, R, $R^4$ und X die oben angegebenen Bedeutungen haben,
mit Phosgen gegebenenfalls in Gegenwart eines Säureakzeptors und gegebenenfalls in Gegenwart eines Verdünnungsmittels umsetzt, oder wenn man

(d) Verbindungen der allgemeinen Formel (VI)

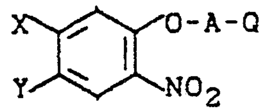

(VI)

in welcher
A, X und Y die oben angegebenen Bedeutungen haben und
Q für Cyano, Carboxy oder $C_1$-$C_4$-Alkoxy-carbonyl steht,
mit einem Reduktionsmittel, gegebenenfalls in Gegenwart eines Katalysators und in Gegenwart eines Verdünnungsmittels umsetzt, oder wenn man

(e) Benzoxazinone der allgemeinen Formel (Ia)

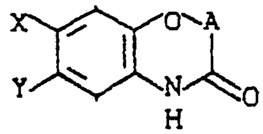

(Ia)

in welcher
A, X und Y die oben angegebenen Bedeutungen haben und
mit Alkylierungsmitteln der allgemeinen Formel (VII)
R - $X^1$ (VII)
in welcher
R die oben angegebene Bedeutung hat und
$X^1$ für Halogen steht,
gegebenenfalls in Gegenwart eines Säureakzeptors und gegebenenfalls in Gegenwart eines Verdünnungsmittels umsetzt, oder wenn man

(f) Benzoxazinone der allgemeinen Formel (Ib)

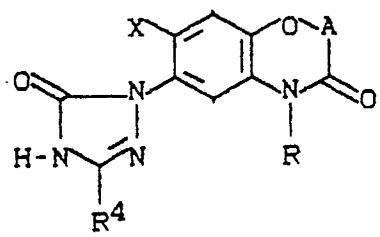

(Ib)

in welcher
A, R, $R^4$ und X die oben angegebenen Bedeutungen haben,
mit Alkylierungsmitteln der allgemeinen Formel (VIII)
$R^5$ - $X^2$ (VIII)
in welcher
$R^5$ für jeweils gegebenenfalls durch Halogen sub stituiertes Alkyl, Alkenyl oder Alkinyl steht und
$X^2$ für Halogen steht,
gegebenenfalls in Gegenwart eines Säureakzeptors und gegebenenfalls in Gegenwart eines Verdünnungsmittels umsetzt, oder wenn man

(g) Benzoxazinone der allgemeinen Formel (IX)

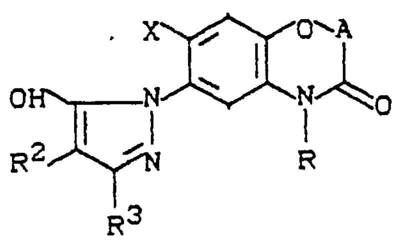

(IX)

in welcher
A, R, $R^2$, $R^3$ und X die oben angegebenen Bedeutungen haben,
mit einem Halogenierungsmittel, gegebenenfalls in Gegenwart eines Katalysators und gegebenenfalls in Gegenwart eines Verdünnungsmittels umsetzt, oder wenn man

(h) Benzoxazinone der allgemeinen Formel (Ic)

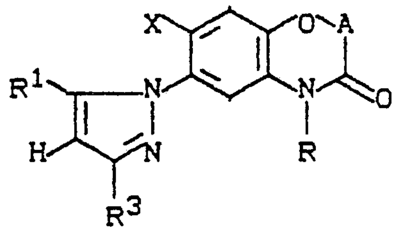

(Ic)

in welcher
A, R, $R^1$, $R^3$ und X die oben angegebenen Bedeutungen haben,
mit einem Halogenierungsmittel oder Nitrierungsmittel, gegebenenfalls in Gegenwart eines Katalysators und gegebenenfalls in Gegenwart eines Verdünnungsmittel, umsetzt.

Schließlich wurde gefunden, daß die neuen substituierten Benzoxazinone der Formel (I) hervorragende herbizide Eigenschaften aufweisen.

Überraschenderweise sind die erfindungsgemäßen substituierten Benzoxazinone der Formel (I) gegen Unkräuter wesentlich stärker wirksam als 5-tert-Butyl-3-(2,4-dichlor-5-isopropoxy-phenyl)-1,3,4-oxadiazol-2-on, welches ein strukturell ähnlicher vorbekannter Wirkstoff gleicher Wirkungsrichtung ist.

Die Kohlenstoffketten in den einzelnen Resten, wie beispielsweise Alkyl, Alkoxy, Alkylthio, Alkoxycarbonyl oder Alkylaminocarbonyl sind jeweils geradkettig oder verzweigt.

Die Erfindung betrifft vorzugsweise Verbindungen der Formel (I), in welcher A

für Methylen ($-CH_2-$), Ethyliden ($-CH(CH_3)-$) oder Isopropyliden ($-C(CH_3)_2-$) steht,

R für Wasserstoff, für gegebenenfalls durch Fluor, Chlor, Brom, Iod, Cyano, Carboxy, Carbamoyl, $C_1$-$C_4$-Alkoxy, $C_1$-$C_4$-Alkoxy-$C_1$-$C_2$-alkoxy, $C_1$-$C_4$-Alkoxy-carbonyl, $C_1$-$C_4$-Alkylamino-carbonyl, $C_1$-$C_4$-Alkoxy-carbonyloxy, $C_1$-$C_4$-Alkoxy-carbonylamino, Aminocarbonyloxy, Aminocarbonylamino und/oder $C_1$-$C_4$-Alkylamino-carbonylamino substituiertes $C_1$-$C_8$-Alkyl, für gegebenenfalls durch Fluor, Chlor, Brom, Iod, Cyano, Carboxy, Carbamoyl und/oder $C_1$-$C_4$-Alkoxy-carbonyl substituiertes $C_3$-$C_8$-Alkenyl, für gegebenenfalls durch Fluor, Chlor, Brom, Iod, Cyano und/oder $C_1$-$C_4$-Alkoxy-carbonyl substituiertes $C_3$-$C_8$-Alkinyl, für gegebenenfalls durch Fluor, Chlor, Brom, Iod und/oder $C_1$-$C_4$-Alkyl substituiertes $C_3$-$C_6$-Cycloalkyl, für gegebenenfalls durch Fluor, Chlor, Brom, Iod und/oder $C_1$-$C_4$-Alkyl substituiertes $C_3$-$C_6$-Cycloalkyl-$C_1$-$C_4$-alkyl, für gegebenenfalls durch Fluor, Chlor, Brom, Cyano, $C_1$-$C_4$-Alkyl, $C_1$-$C_4$-Alkoxy und/oder $C_1$-$C_4$-Alkoxy-carbonyl substituiertes Benzyl oder für gegebenenfalls durch Fluor, Chlor, Brom, Iod, Cyano, $C_1$-$C_4$-Alkyl und/oder $C_1$-$C_4$-Alkoxy substituiertes Heteroarylalkyl aus der Reihe Furyl-$C_1$-$C_4$-alkyl, Thienyl-$C_1$-$C_4$-alkyl, Oxazolyl-$C_1$-$C_4$-alkyl, Oxadiazolyl-$C_1$-$C_4$-alkyl, Thiazolyl-$C_1$-$C_4$-alkyl, Thiadiazolyl-$C_1$-$C_4$-alkyl, Pyridinyl-$C_1$-$C_4$-al kyl und Pyrimidinyl-$C_1$-$C_4$-alkyl steht,
X für Wasserstoff, Fluor oder Chlor steht und
Y für die nachstehenden heterocyclischen Gruppierungen steht

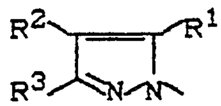

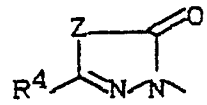

worin
$R^1$ für Wasserstoff, Fluor, Chlor, Brom oder für gegebenenfalls durch Fluor und/oder Chlor substituiertes $C_1$-$C_4$-Alkyl steht,
$R^2$ für Wasserstoff, Cyano, Nitro, Fluor, Chlor, Brom, Iod oder für gegebenenfalls durch Fluor und/oder Chlor substituiertes $C_1$-$C_4$-Alkyl steht,
$R^3$ für Wasserstoff oder gegebenenfalls durch Fluor und/oder Chlor substituiertes $C_1$-$C_4$-Alkyl steht oder zusammen mit $R^2$ für $C_3$- oder $C_4$-Alkandiyl steht,
$R^4$ für Wasserstoff oder für jeweils gegebenenfalls durch Fluor, Chlor und/oder Brom substituiertes $C_1$-$C_4$-Alkyl, $C_3$-$C_6$-Cycloalkyl, $C_2$-$C_4$-Alkenyl oder $C_2$-$C_4$-Alkinyl steht, und
Z für Sauerstoff oder für die Gruppierung -N-$R^5$ steht, worin
$R^5$ für Wasserstoff oder für jeweils gegebenenfalls durch Fluor und/oder Chlor substituiertes $C_1$-$C_4$-Alkyl, $C_3$-$C_4$-Alkenyl oder $C_3$-$C_4$-Alkinyl steht, oder zusammen mit $R^4$ mit $C_3$-oder $C_4$-Alkandiyl steht.

Die Erfindung betrifft insbesondere Verbindungen der Formel (I), in welcher
A für Methylen (-$CH_2$-) oder Ethyliden

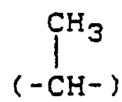

steht,
R für Wasserstoff, für gegebenenfalls durch Fluor, Chlor, Cyano oder $C_1$-$C_4$-Alkoxy substituiertes $C_1$-$C_4$-Alkyl, für $C_1$-$C_4$-Alkoxy-carbonyl-$C_1$-$C_2$-alkyl, für $C_1$-$C_4$-Alkylamino-carbonyl-$C_1$-$C_2$-alkyl, für $C_3$-$C_6$-Alkenyl, für $C_3$-$C_4$-Alkinyl, für Benzyl oder Pyridinylmethyl steht,
X für Wasserstoff oder Fluor steht und
Y für die nachstehenden heterocyclischen Gruppierungen steht

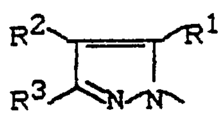

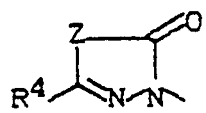

worin
$R^1$ für Wasserstoff, Chlor, Brom, $C_1$-$C_4$-Alkyl oder durch Fluor und/oder Chlor substituiertes Methyl steht,
$R^2$ für Wasserstoff, Cyano, Nitro, Chlor, Brom, $C_1$-$C_4$-Alkyl oder durch Fluor und/oder Chlor substituiertes Methyl steht,
$R^3$ für Wasserstoff, $C_1$-$C_4$-Alkyl oder durch Fluor und/oder Chlor substituiertes Methyl steht oder zusammen mit $R^2$ für $C_3$- oder $C_4$-Alkandiyl steht,
$R^4$ für Wasserstoff oder für jeweils gegebenenfalls durch Fluor und/oder Chlor substituiertes $C_1$-$C_4$-Alkyl, $C_3$-$C_6$-Cycloalkyl, $C_2$-$C_4$-Alkenyl oder $C_2$-$C_4$-Alkinyl steht, und
Z für Sauerstoff oder die Gruppierung -N-$R^5$ steht, worin
$R^5$ für Wasserstoff, $C_1$-$C_2$-Alkyl, jeweils durch Fluor und/oder Chlor substituiertes Methyl, $C_3$-$C_4$-Alkenyl oder $C_3$-$C_4$-Alkinyl steht.

Beispiele für die erfindungsgemäßen Verbindungen der Formel (I) sind in der nachstehenden Tabelle 1 aufgeführt - vgl. auch die Herstellungsbeispiele.

<u>Tabelle 1</u>: Beispiele für die Verbindungen der Formel (I)

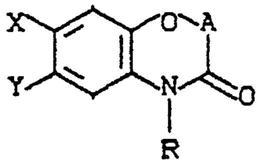

(I)

| A | R | X | Y |
|---|---|---|---|
| $CH_2$ | H | H | 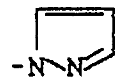 |
| $CH_2$ | H | F | 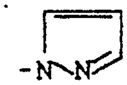 |
| $CH_2$ | H | Cl | 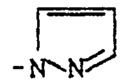 |
| $CH_2$ | $CH_3$ | F | 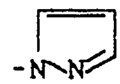 |
| $CH_2$ | $-CH_2CH=CH_2$ | F | 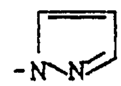 |
| $CH_2$ | $-CH_2C{\equiv}CH$ | F | 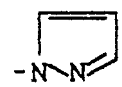 |
| $CH_2$ | $C_2H_5$ | F | 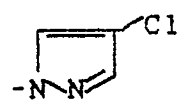 |
| $CH_2$ | $-CH_2CN$ | F | 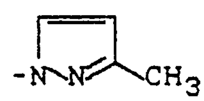 |
| $CH_2$ | $-CH_2OCH_3$ | F | 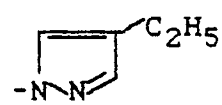 |

Tabelle 1 - Fortsetzung

| A | R | X | Y |
|---|---|---|---|
| $CH_2$ | $-CH_2$ 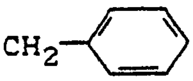 | F | 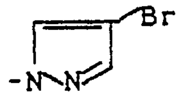 |
| $CH_2$ | $-CH_2$ 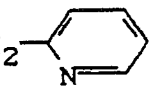 | F | 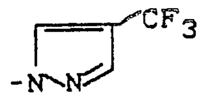 |
| $CH_2$ | $C_3H_7$ | F | 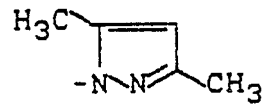 |
| $CH_2$ | $C_4H_9$ | F | 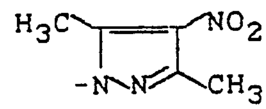 |
| $CH_2$ | H | F | 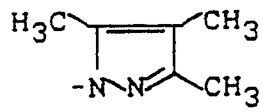 |
| $CH_2$ | $-CH_2OCH_3$ | F | 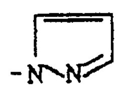 |
| $CH_2$ | $-CH_2C{\equiv}CH$ | F | 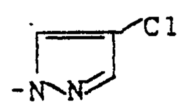 |
| $CH_2$ | $-CH_2C{\equiv}CH$ | F | 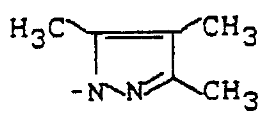 |
| $CH_2$ | $-CH_2CN$ | F | 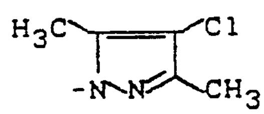 |
| $CH_2$ | $-CH_2OCH_3$ | F | 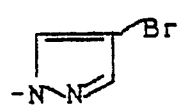 |
| $CH_2$ | $-CH_2OC_2H_5$ | F | 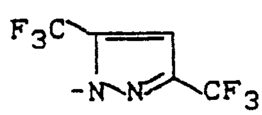 |

Tabelle 1 - Fortsetzung

| A | R | X | Y |
|---|---|---|---|
| $CH_2$ | $-CH_2CH=CH_2$ | F | 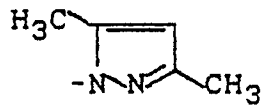 |
| $CH_2$ | $-CH_2CH(CH_3)_2$ | H | 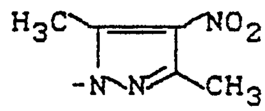 |
| $CH_2$ | $-CH_2C\equiv CH$ | F | 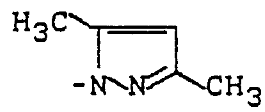 |
| $CH_2$ | H | H | 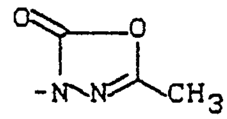 |
| $CH_2$ | $CH_3$ | H | 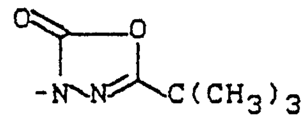 |
| $CH_2$ | $C_2H_5$ | F | 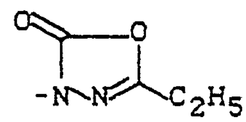 |
| $CH_2$ | $C_3H_7$ | F | 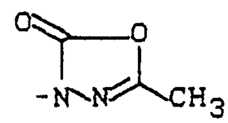 |
| $CH_2$ | $CH(CH_3)_2$ | F | 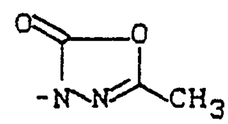 |
| $CH_2$ | $C_4H_9$ | H | 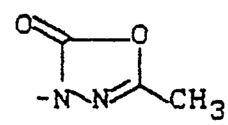 |
| $CH_2$ | $-\underset{\vert \atop CH_3}{CH}-CH_2CH_3$ | H | 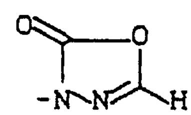 |

Tabelle 1 - Fortsetzung

| A | R | X | Y |
|---|---|---|---|
| $CH_2$ | $-CH_2CH=CH_2$ | F | 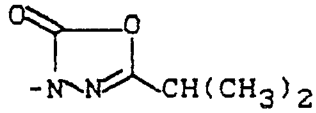 |
| $CH_2$ | $-CH_2C\equiv CH$ | F | 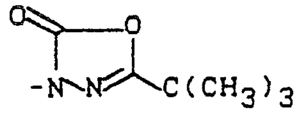 |
| $CH_2$ | $-CH_2C\equiv C-CH_3$ | F | 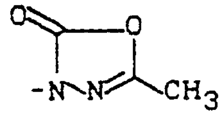 |
| $CH_2$ | $-CH_2CH=CH-CH_3$ | F | 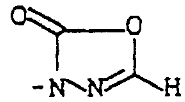 |
| $CH_2$ | $-CH_2COOCH_3$ | F | 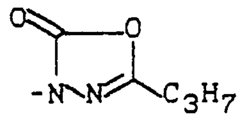 |
| $CH_2$ | $-CH_2COOC_2H_5$ | H | 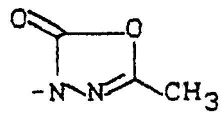 |
| $CH_2$ | $-CH(CH_3)-COOCH_3$ | F | 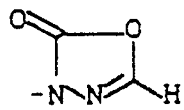 |
| $CH_2$ | $-CH_2CONHCH_3$ | H | 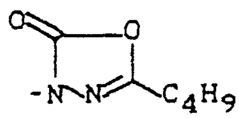 |
| $CH_2$ | $-CH_2CONHC_4H_9$ | H | 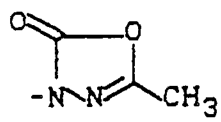 |
| $CH_2$ | $-CH(CH_3)-CONHC_2H_5$ | H | 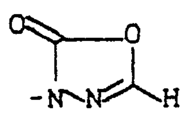 |

xL1,0

Tabelle 1 - Fortsetzung

| A | R | X | Y |
| --- | --- | --- | --- |
| $CH_2$ | $-\underset{CH_3}{CH}CH_2OCH_3$ | F | 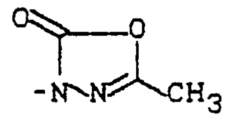 |
| $CH_2$ | $-\underset{CH_3}{CH}CH_2-OC_2H_5$ | F | 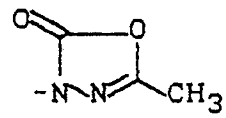 |
| $CH_2$ | $-CH_2CH_2OCH_3$ | F | 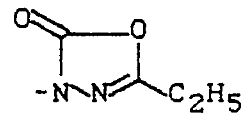 |
| $CH_2$ | $-CH_2CH_2OC_2H_5$ | F | 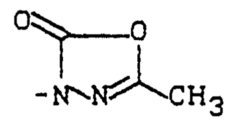 |
| $CH_2$ | $-CH_2CN$ | F | 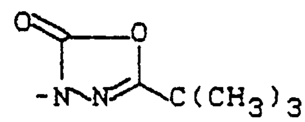 |
| $CH_2$ | $-\underset{CH_3}{CH}CN$ | F | 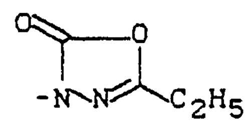 |
| $CH_2$ | H | H | 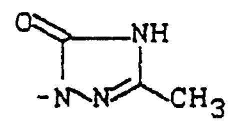 |
| $CH_2$ | $CH_3$ | F | 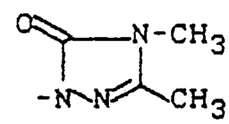 |
| $CH_2$ | $C_2H_5$ | F | 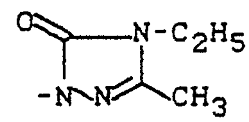 |
| $CH_2$ | $-CH_2CH=CH_2$ | F | 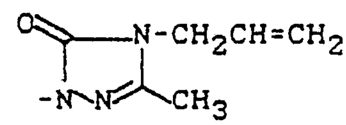 |

Tabelle 1 - Fortsetzung

| A | R | X | Y |
|---|---|---|---|
| $CH_2$ | $-CH_2C{\equiv}CH$ | F | 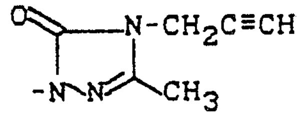 |
| $CH_2$ | $-CHF_2$ | F | 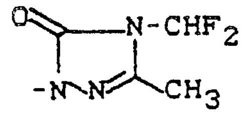 |
| $CH_2$ | $-CH_2C{\equiv}CH$ | F | 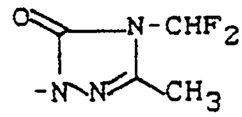 |
| $CH_2$ | H | H | 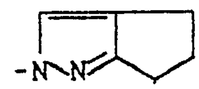 |
| $CH_2$ | H | F | 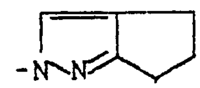 |
| $CH_2$ | H | F | 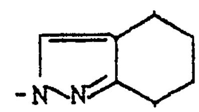 |
| $CH_2$ | $CH_3$ | F | 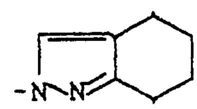 |
| $CH_2$ | $-CH_2CH{=}CH_2$ | F | 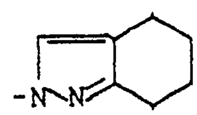 |
| $CH_2$ | $-CH_2C{\equiv}CH$ | F | 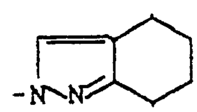 |
| $CH_2$ | $-CH_2OCH_3$ | F | 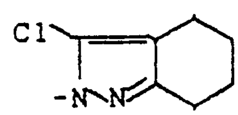 |

Tabelle 1 - Fortsetzung

| A | R | X | Y |
|---|---|---|---|
| $CH_2$ | $-CH_2CN$ | F | 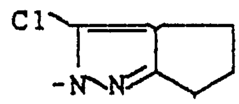 |
| $CH_2$ | $-CH_2C{\equiv}CH$ | F | 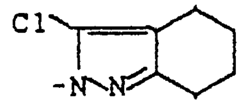 |
| $CH_2$ | $-CH_2CH_2CN$ | F | 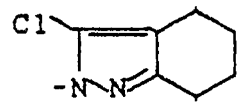 |
| $CH_2$ | $-CH_2-CH{=}CH_2$ | F | 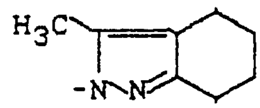 |
| $CH_2$ | $-CH_2C{\equiv}CH$ | F | 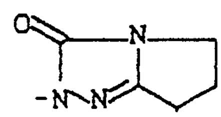 |
| $CH_2$ | $-CH_2CN$ | F | 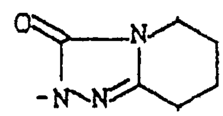 |
| $CH_2$ | $-CH_2CH{=}CH_2$ | F | 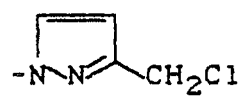 |
| $CH_2$ | $-CH_2C{\equiv}CH$ | F | 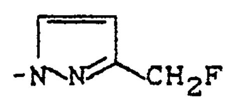 |
| $CH_2$ | $-CH_2C{\equiv}CH$ | F | 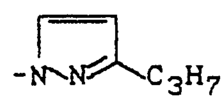 |
| $CH_2$ | $-CH_2CN$ | F | 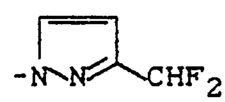 |

Tabelle 1 - Fortsetzung

| A | R | X | Y |
|---|---|---|---|
| $CH_2$ | H | F | 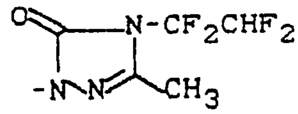 |
| $CH_2$ | $-CF_2CHF_2$ | F | 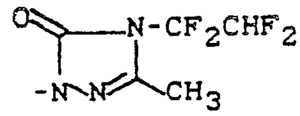 |
| $CH_2$ | H | F | 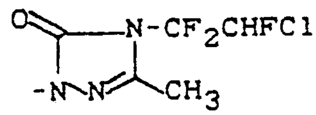 |
| $CH_2$ | $-CF_2CHFCl$ | F | 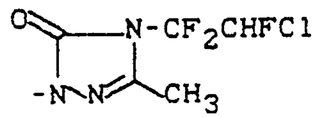 |
| $CH_2$ | H | F | 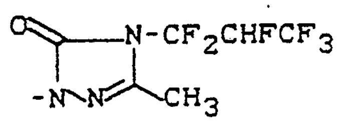 |
| $CH_2$ | $-CF_2CHFCF_3$ | F | 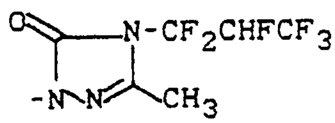 |
| $CH_2$ | H | F | 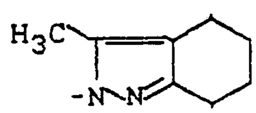 |
| $CH_2$ | H | F | 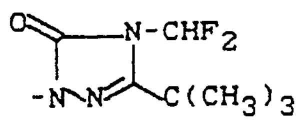 |
| $CH_2$ | $-CH_2-COOC_2H_5$ | F | 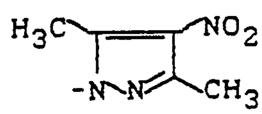 |
| $CH_2$ | $-CH-COOC_2H_5$<br>$\vert$<br>$C_2H_5$ | F | 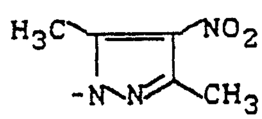 |

Tabelle 1 - Fortsetzung

| A | R | X | Y |
|---|---|---|---|
| $CH_2$ | $-CH(CH_3)-COOC_2H_5$ | F | 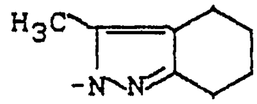 |
| $CH_2$ | 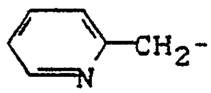 | F | 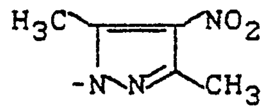 |
| $CH_2$ | $-CH(CH_3)-COOC_2H_5$ | F | 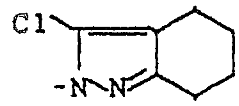 |
| $CH_2$ | $-CH_2-CH=CH-Cl$ | F | 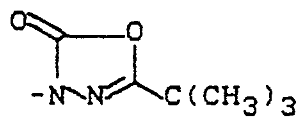 |
| $CH_2$ | $-CH_2-CH=CHCl$ | F | 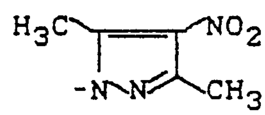 |
| $CH_2$ | $-CH_2-CH=CHBr$ | F | 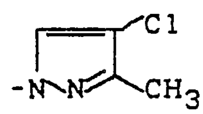 |
| $CH_2$ | $-CH_2-CH=CH-Cl$ | F | 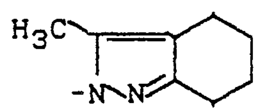 |
| $CH_2$ | $-CH_2-CH=CH-CH_3$ | F | 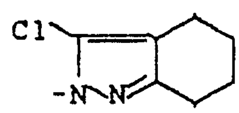 |
| $CH_2$ | $-CH_2-CH=CH-Cl$ | F | 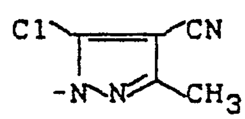 |
| $CH_2$ | $-CH_2-CH=CH-CH_3$ | F | 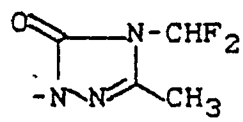 |

Tabelle 1 - Fortsetzung

| A | R | X | Y |
|---|---|---|---|
| $CH_2$ | $-CH_2-CH=CH-Cl$ | F | 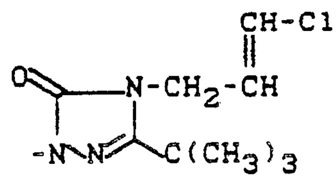 |
| $CH_2$ | $CH_3$ | F | 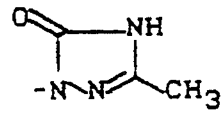 |
| $CH_2$ | H | F | 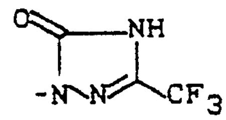 |
| $CH_2$ | H | F | 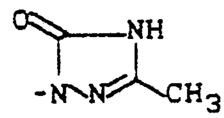 |
| $CH_2$ | $-CH_2C\equiv CH$ | F | 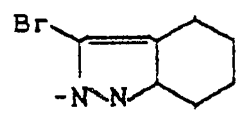 |
| $CH_2$ | $-CH_2CH=CH_2$ | F | 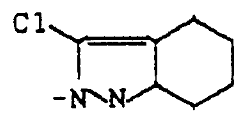 |
| $CH_2$ | $-CH_2COOC_2H_5$ | F | 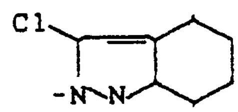 |
| $CH_2$ | $-CH_2CH_2OC_2H_5$ | F | 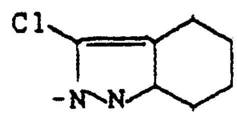 |
| $CHCH_3$ | H | F | 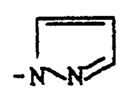 |
| $CHCH_3$ | $CH_3$ | F | 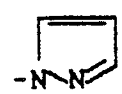 |

<u>Tabelle 1</u> - Fortsetzung

| A | R | X | Y |
|---|---|---|---|
| $CHCH_3$ | $-CH_2CH=CH_2$ | F | 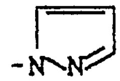 |
| $CHCH_3$ | $-CH_2C\equiv CH$ | F | 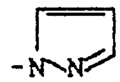 |
| $CHCH_3$ | $C_2H_5$ | F | 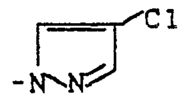 |
| $CHCH_3$ | $-CH_2CN$ | F | 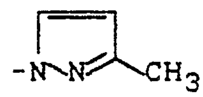 |
| $CHCH_3$ | $-CH_2OCH_3$ | F | 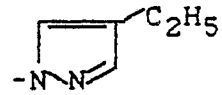 |
| $CHCH_3$ | 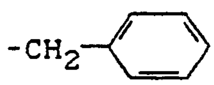 | F | 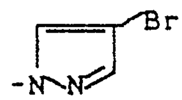 |
| $CHCH_3$ | 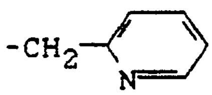 | F | 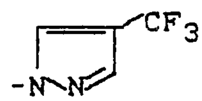 |
| $CHCH_3$ | $C_3H_7$ | F | 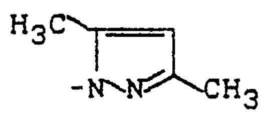 |
| $CHCH_3$ | $C_4H_9$ | F | 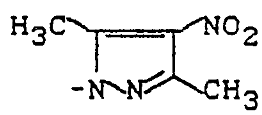 |
| $CHCH_3$ | H | F | 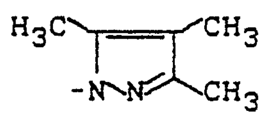 |

Tabelle 1 - Fortsetzung

| A | R | X | Y |
|---|---|---|---|
| $CHCH_3$ | $-CH_2OCH_3$ | F | 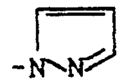 |
| $CHCH_3$ | $-CH_2C{\equiv}CH$ | F | 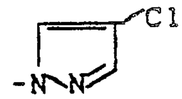 |
| $CHCH_3$ | $-CH_2C{\equiv}CH$ | F | 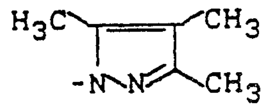 |
| $CHCH_3$ | $-CH_2CN$ | F | 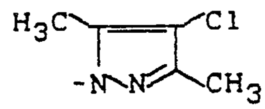 |
| $CHCH_3$ | $-CH_2OCH_3$ | F | 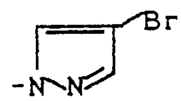 |
| $CHCH_3$ | $-CH_2OC_2H_5$ | F | 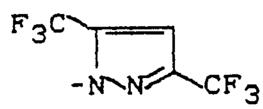 |
| $CHCH_3$ | $-CH_2CH{=}CH_2$ | F | 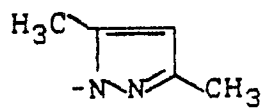 |
| $CHCH_3$ | $-CH_2CH(CH_3)_2$ | H | 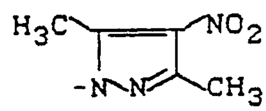 |
| $CHCH_3$ | $-CH_2C{\equiv}CH$ | F | 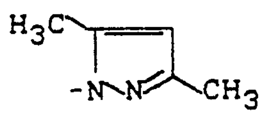 |
| $CHCH_3$ | H | H | 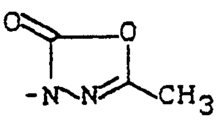 |

Tabelle 1 - Fortsetzung

| A | R | X | Y |
|---|---|---|---|
| $CHCH_3$ | $CH_3$ | H | 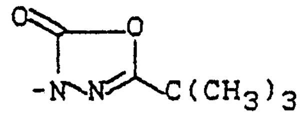 |
| $CHCH_3$ | $C_2H_5$ | F | 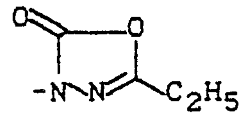 |
| $CHCH_3$ | $C_3H_7$ | F | 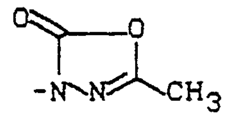 |
| $CHCH_3$ | $CH(CH_3)_2$ | F | 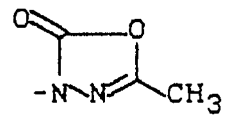 |
| $CHCH_3$ | $C_4H_9$ | H | 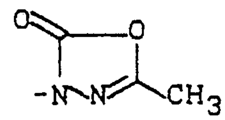 |
| $CHCH_3$ | $-CH(CH_3)-CH_2CH_3$ | H | 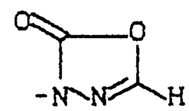 |
| $CHCH_3$ | $-CH_2CH=CH_2$ | F | 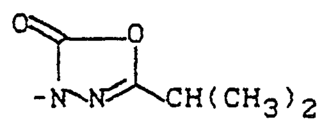 |
| $CHCH_3$ | $-CH_2C\equiv CH$ | F | 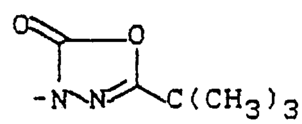 |
| $CHCH_3$ | $-CH_2C\equiv C-CH_3$ | F | 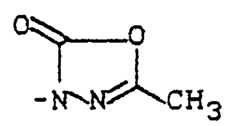 |
| $CHCH_3$ | $-CH_2CH=CH-CH_3$ | F | 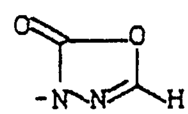 |

Tabelle 1 - Fortsetzung

| A | R | X | Y |
|---|---|---|---|
| $CHCH_3$ | $-CH_2COOCH_3$ | F | 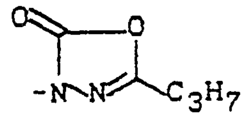 |
| $CHCH_3$ | $-CH_2COOC_2H_5$ | H | 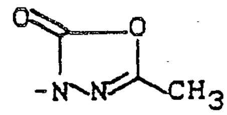 |
| $CHCH_3$ | $-CH(CH_3)-COOCH_3$ | F | 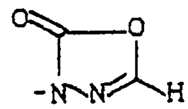 |
| $CHCH_3$ | $-CH_2CONHCH_3$ | H | 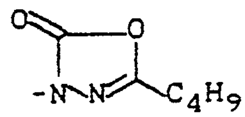 |
| $CHCH_3$ | $-CH_2CONHC_4H_9$ | H | 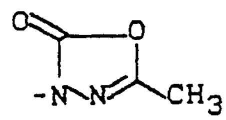 |
| $CHCH_3$ | $-CH(CH_3)-CONHC_2H_5$ | H | 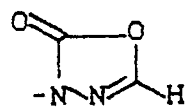 |
| $CHCH_3$ | $-CH(CH_3)CH_2OCH_3$ | F | 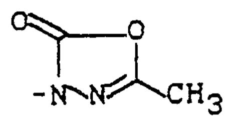 |
| $CHCH_3$ | $-CH(CH_3)CH_2-OC_2H_5$ | F | 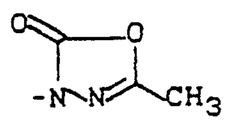 |
| $CHCH_3$ | $-CH_2CH_2OCH_3$ | F | 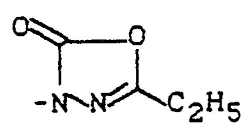 |

Tabelle 1 - Fortsetzung

| A | R | X | Y |
|---|---|---|---|
| $CHCH_3$ | $-CH_2CH_2OC_2H_5$ | F | 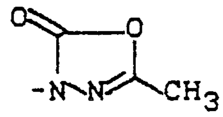 |
| $CHCH_3$ | $-CH_2CN$ | F | 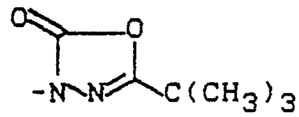 |
| $CHCH_3$ | $-CHCN$<br>\|<br>$CH_3$ | F | 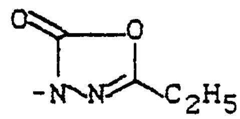 |
| $CHCH_3$ | H | H | 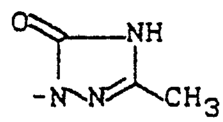 |
| $CHCH_3$ | $CH_3$ | F | 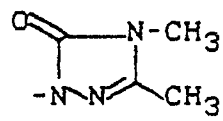 |
| $CHCH_3$ | $C_2H_5$ | F | 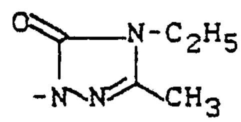 |
| $CHCH_3$ | $-CH_2CH=CH_2$ | F | 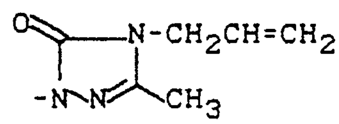 |
| $CHCH_3$ | $-CH_2C\equiv CH$ | F | 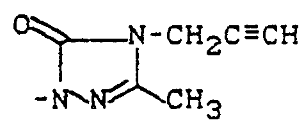 |
| $CHCH_3$ | $-CHF_2$ | F | 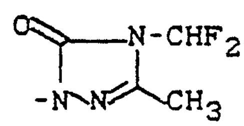 |
| $CHCH_3$ | $-CH_2C\equiv CH$ | F | 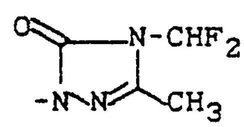 |

Tabelle 1 - Fortsetzung

| A | R | X | Y |
|---|---|---|---|
| $CHCH_3$ | H | H | 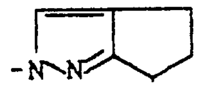 |
| $CHCH_3$ | H | F | 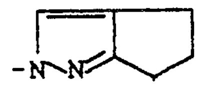 |
| $CHCH_3$ | H | F | 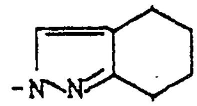 |
| $CHCH_3$ | $CH_3$ | F | 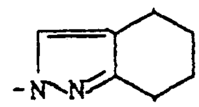 |
| $CHCH_3$ | $-CH_2CH=CH_2$ | F | 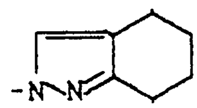 |
| $CHCH_3$ | $-CH_2C{\equiv}CH$ | F | 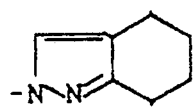 |
| $CHCH_3$ | $-CH_2OCH_3$ | F | 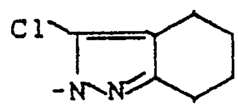 |
| $CHCH_3$ | $-CH_2CN$ | F | 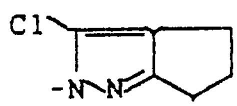 |
| $CHCH_3$ | $-CH_2C{\equiv}CH$ | F | 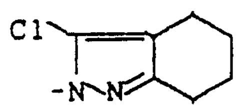 |
| $CHCH_3$ | $-CH_2CH_2CN$ | F | 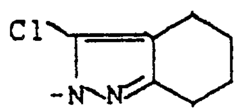 |

Tabelle 1 - Fortsetzung

| A | R | X | Y |
|---|---|---|---|
| $CHCH_3$ | $-CH_2-CH=CH_2$ | F | 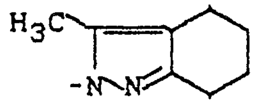 |
| $CHCH_3$ | $-CH_2C{\equiv}CH$ | F | 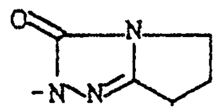 |
| $CHCH_3$ | $-CH_2CN$ | F | 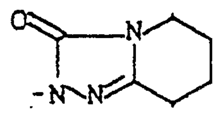 |
| $CHCH_3$ | $-CH_2CH=CH_2$ | F | 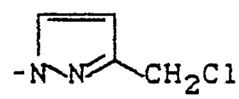 |
| $CHCH_3$ | $-CH_2C{\equiv}CH$ | F | 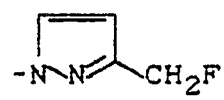 |
| $CHCH_3$ | $-CH_2C{\equiv}CH$ | F | 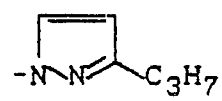 |
| $CHCH_3$ | $-CH_2CN$ | F | 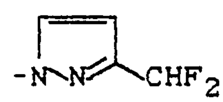 |
| $CHCH_3$ | H | F | 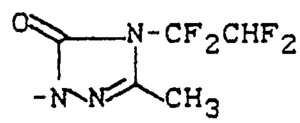 |
| $CHCH_3$ | $-CF_2CHF_2$ | F | 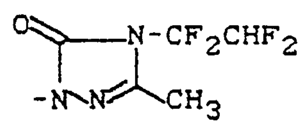 |
| $CHCH_3$ | H | F | 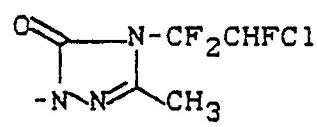 |

Tabelle 1 - Fortsetzung

| A | R | X | Y |
|---|---|---|---|
| $CHCH_3$ | $-CF_2CHFCl$ | F | 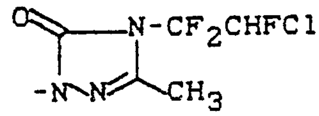 |
| $CHCH_3$ | H | F | 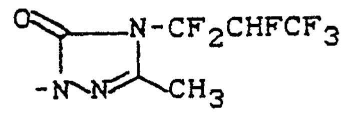 |
| $CHCH_3$ | $-CF_2CHFCF_3$ | F | 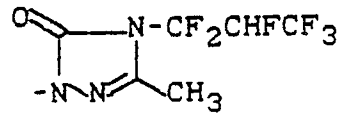 |
| $CHCH_3$ | H | F | 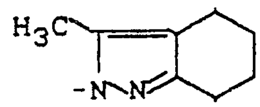 |
| $CHCH_3$ | H | F | 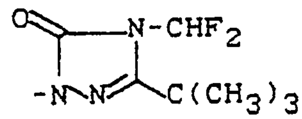 |
| $CHCH_3$ | $-CH_2-COOC_2H_5$ | F | 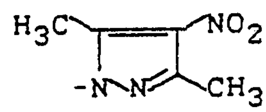 |
| $CHCH_3$ | $-CH-COOC_2H_5$<br>\|<br>$C_2H_5$ | F | 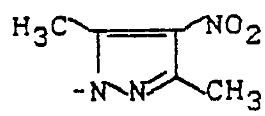 |
| $CHCH_3$ | $-CH-COOC_2H_5$<br>\|<br>$CH_3$ | F | 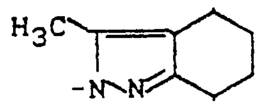 |
| $CHCH_3$ | 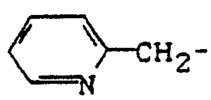 | F | 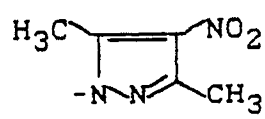 |
| $CHCH_3$ | $-CH-COOC_2H_5$<br>\|<br>$CH_3$ | F | 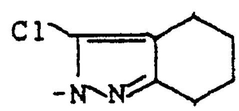 |

Tabelle 1 - Fortsetzung

| A | R | X | Y |
|---|---|---|---|
| $CHCH_3$ | $-CH_2-CH=CHCl$ | F | 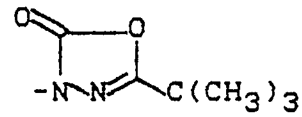 |
| $CHCH_3$ | $-CH_2-CH=CHCl$ | F | 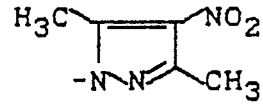 |
| $CHCH_3$ | $-CH_2-CH=CHBr$ | F | 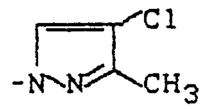 |
| $CHCH_3$ | $-CH_2-CH=CH-Cl$ | F | 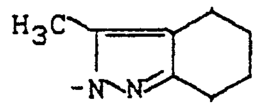 |
| $CHCH_3$ | $-CH_2-CH=CH-CH_3$ | F | 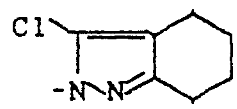 |
| $CHCH_3$ | $-CH_2-CH=CH-Cl$ | F | 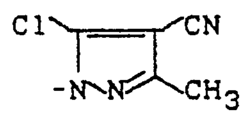 |
| $CHCH_3$ | $-CH_2-CH=CH-CH_3$ | F | 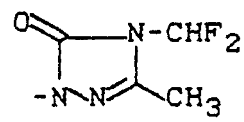 |
| $CHCH_3$ | $-CH_2-CH=CH-Cl$ | F | 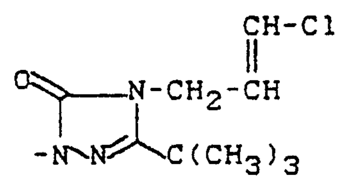 |
| $CHCH_3$ | $CH_3$ | F | 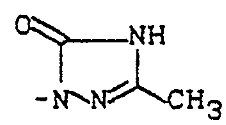 |
| $CHCH_3$ | H | F | 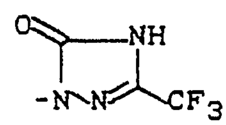 |

Tabelle 1 - Fortsetzung

| A | R | X | Y |
|---|---|---|---|
| $CHCH_3$ | H | F | 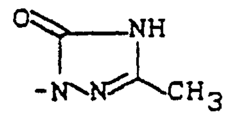 |
| $CHCH_3$ | $-CH_2C{\equiv}CH$ | F | 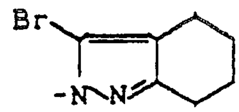 |
| $CHCH_3$ | $-CH_2CH{=}CH_2$ | F | 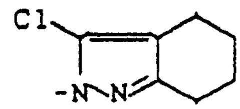 |
| $CHCH_3$ | $-CH_2COOC_2H_5$ | F | 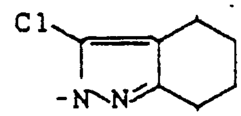 |
| $CHCH_3$ | $-CH_2CH_2OC_2H_5$ | F | 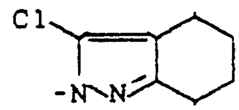 |
| $CH_2$ | $-CH_2-C{\equiv}CH$ | F | 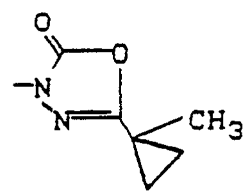 |
| $CH_2$ | $-CH_2CH{=}CH_2$ | F | 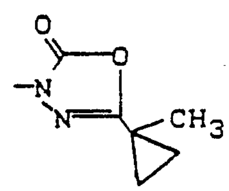 |
| $CH_2$ | $-CH_2CN$ | F | 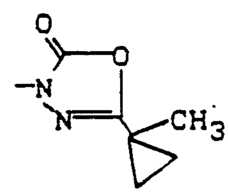 |

<u>Tabelle 1</u> - Fortsetzung

| A | R | X | Y |
|---|---|---|---|
| $CH_2$ | $-CHCOOC_2H_5$<br>$\vert$<br>$CH_3$ | F | 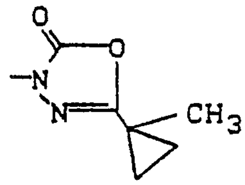 |
| $CH_2$ | 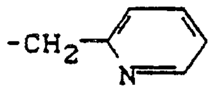 | F | 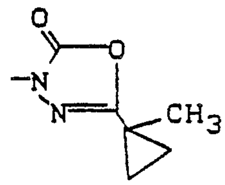 |
| $CHCH_3$ | $-CH_2-C\equiv CH$ | F | 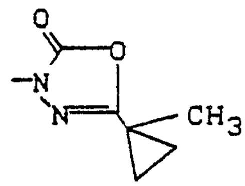 |
| $CHCH_3$ | $-CH_2CH=CH_2$ | F | 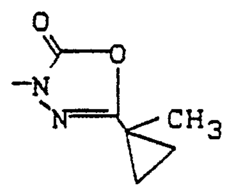 |
| $CHCH_3$ | $-CH_2CN$ | F | 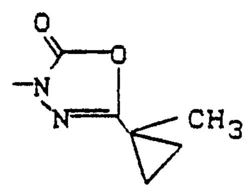 |
| $CHCH_3$ | $-CHCOOC_2H_5$<br>$\vert$<br>$CH_3$ | F | 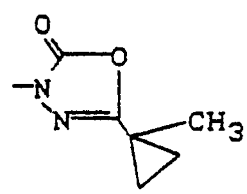 |

Tabelle 1 - Fortsetzung

| A | R | X | Y |
|---|---|---|---|
| $CHCH_3$ | $-CH_2$ 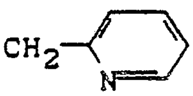 | F | 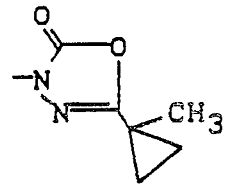 |
| $CH_2$ | $-CH_2C\equiv CH$ | F | 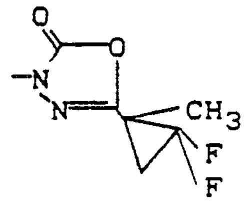 |
| $CH_2$ | $-CH_2C\equiv CH$ | H | 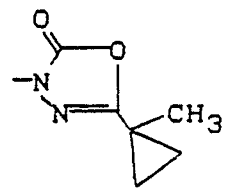 |
| $CH_2$ | $-CH_2CH=CH_2$ | H | 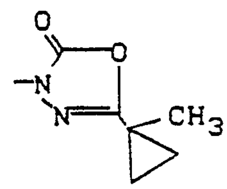 |
| $CH_2$ | $-CH_2CN$ | H | 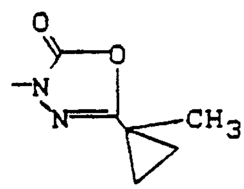 |
| $CH_2$ | $-\underset{CH_3}{\underset{\vert}{C}}HCOOC_2H_5$ | H | 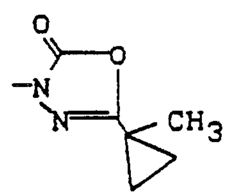 |

<u>Tabelle 1</u> - Fortsetzung

| A | R | X | Y |
|---|---|---|---|
| $CH_2$ | $-CH_2$ 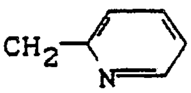 | H | 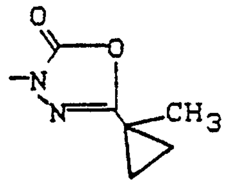 |
| $CHCH_3$ | $-CH_2C{\equiv}CH$ | H | 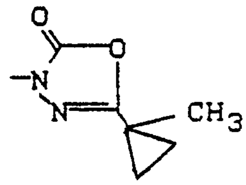 |
| $CHCH_3$ | $-CH_2CH{\equiv}CH_2$ | H | 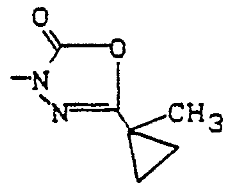 |
| $CHCH_3$ | $-CH_2CN$ | H | 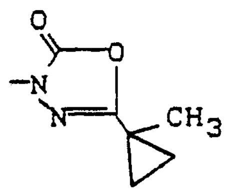 |
| $CHCH_3$ | $-CHCOOC_2H_5$<br>\|<br>$CH_3$ | H | 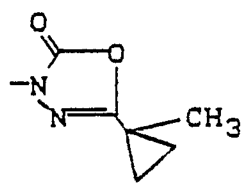 |
| $CHCH_3$ | $-CH_2$ 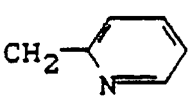 | H | 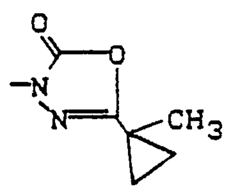 |
| $CH_2$ | $-CH_2C{\equiv}CH$ | H | 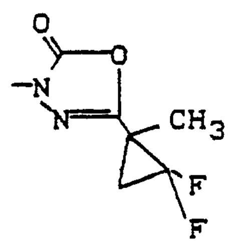 |

Verwendet man für das erfindungsgemäße Verfahren (a) beispielsweise 7-Fluor-6-hydrazino-3,4-dihydro-3-oxo-2H-1,4-benzoxazin und Acetylaceton als Ausgangsstoffe, so kann der Reaktionsablauf durch das folgenden Formelschema wiedergegeben werden:

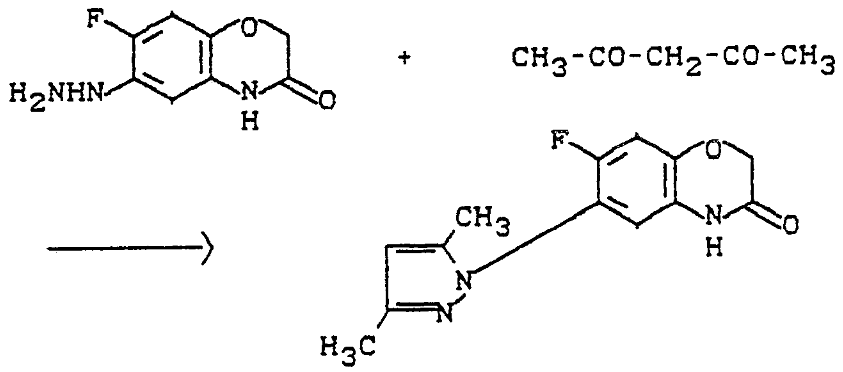

Verwendet man für das erfindungsgemäße Verfahren (b) beispielsweise 6-Hydrazino-3,4-dihydro-3-oxo-2H-1,4-benzoxazin und N-Methoxycarbonyl-O-ethyl-acetimidester als Ausgangsstoffe, so kann der Reaktionsablauf durch das folgende Formelschema wiedergegeben werden:

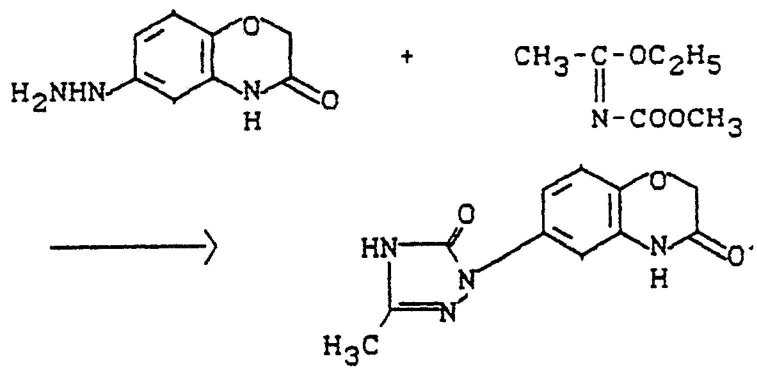

Verwendet man für das erfindungsgemäße Verfahren (c) beispielsweise 6-Acetylhydrazino-7-chlor-3,4-dihydro-3-oxo-2H-1,4-benzoxazin und Phosgen als Ausgangsstoffe so kann der Reaktionsablauf durch das folgende Formelschema wiedergegeben werden:

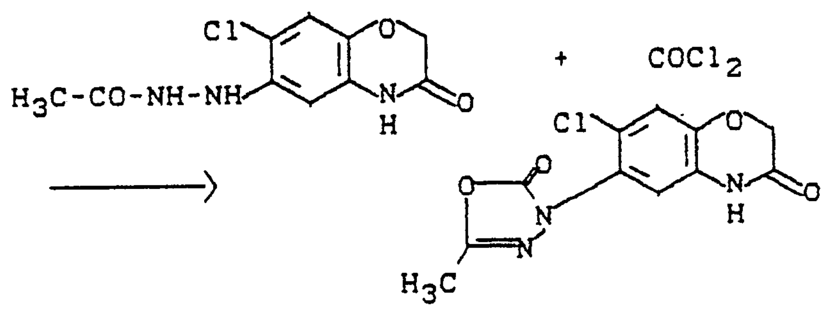

Verwendet man für das erfindungsgemäße Verfahren (d) beispielsweise 1-(2-Fluor-4-methoxycarbonyl-methoxy-5-nitro-phenyl)-pyrazol und Wasserstoff als Ausgangsstoffe, so kann der Reaktionsablauf durch das folgende Formelschema wiedergegeben werden:

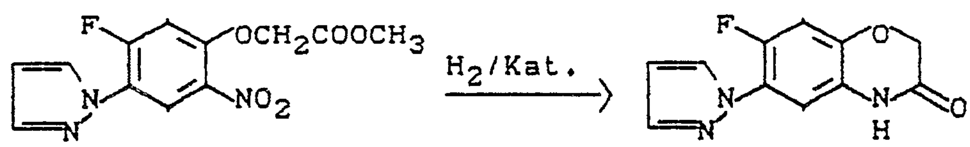


Verwendet man für das erfindungsgemäße Verfahren (e) beispielsweise 7-Fluor-6-trimethylpyrazolyl-3,4-dihydro-3-oxo-2H-1,4-benzoxazin und Allylbromid als Ausgangsstoffe, so kann der Reaktionsablauf durch das folgende Formelschema wiedergegeben werden:

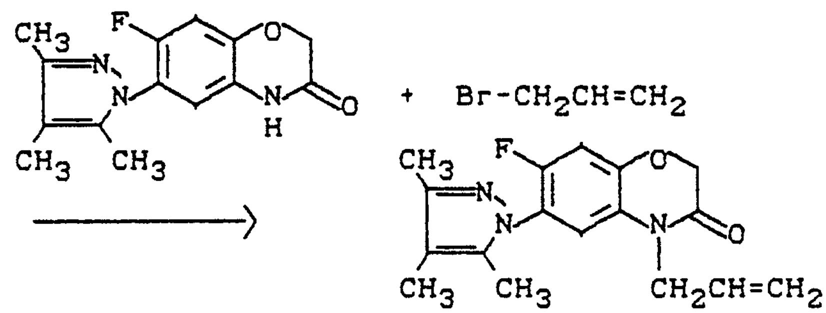


Verwendet man für das erfindungsgemäße Verfahren (f) beispielsweise 7-Fluor-6-(4,5-dihydro-3-methyl-5-oxo-1H-triazol-1-yl)-3,4-dihydro-3-oxo-2H-4-propargyl-1,4-benzoxazin und Allylbromid als Ausgangsstoffe, so kann der Reaktionsablauf durch das folgende Formelschema wiedergegeben werden:

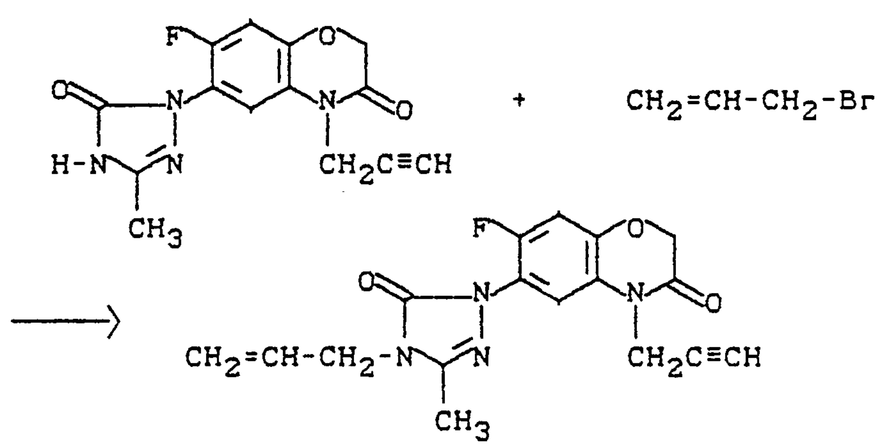


Verwendet man für das erfindungsgemäße Verfahren (g) beispielsweise 7-Fluor-6-(5-hydroxy-3-trifluormethylpyrazol-1-yl)-3,4-dihydro-4-methyl-3-oxo-2H-1,4-benzoxazin und Thionylchlorid als Ausgangsstoffe, so kann der Reaktionsablauf durch das folgende Formelschema wiedergegeben werden:

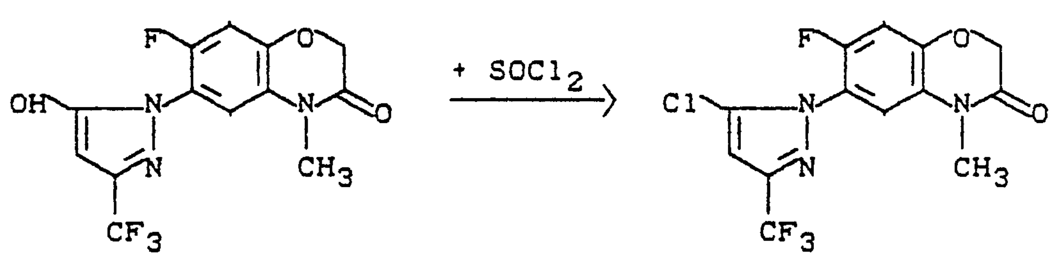


Verwendet man für das erfindungsgemäße Verfahren (h) beispielsweise 7-Fluor-6-(3,5-dimethylpyrazol-1-yl)-3,4- dihydro-4-methoxymethyl-3-oxo-2H-1,4-benzoxazin und Brom als Ausgangsstoffe, so kann der Reaktionsablauf durch das folgende Formelschema wiedergegeben werden:

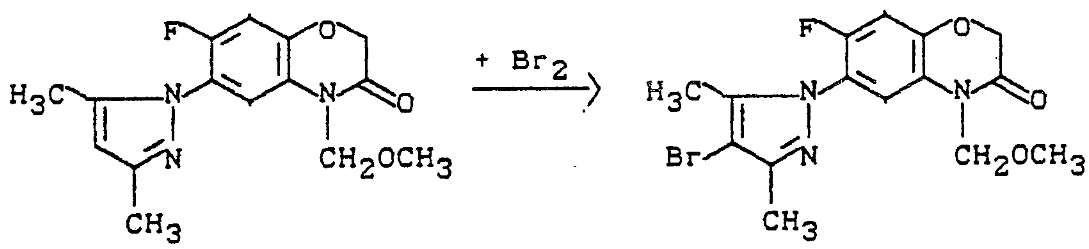


Die beim erfindungsgemäßen Verfahren (a) zur Herstellung von Verbindungen der Formel (I) als Ausgangsstoffe zu verwendenden Hydrazino-benzoxazinone sind durch die Formel (II) allgemein definiert.

In Formel (II) haben A, R und X vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, R und X angegeben wurden.

Als Beispiele für die Ausgangsstoffe der Formel (II) seien genannt:
6-Hydrazino-3,4-dihydro-3-oxo-2H-1,4-benzoxazin, 7-Chlor und 7-Fluor-6-hydrazino-3,4-dihydro-3-oxo-2H-1,4-benzoxazin, 4-Methyl, 4-Ethyl-, 4-Propyl-, 4-Isopropyl-, 4-Butyl-, 4-sec-Butyl-, 4-Isobutyl, 4-Cyanomethyl-, 4-(1-Cyano-ethyl)-, 4-(2-Cyano-ethyl)-, 4-Methoxymethyl-, 4-Ethoxymethyl-, 4-Difluormethyl-, 4-Allyl-, 4-(3-Methyl-2-buten-1-yl)-, 4-(2-Buten-1-yl)-, 4-Propargyl-, 4-(2-Butin-1-yl)-, 4-Methoxycarbonylmethyl-, 4-Ethoxycarbonylmethyl-, 4-(1-Methoxycarbonylethyl)-, 4-(1- Ethoxycarbonyl-ethyl)-, 4-Methylaminocarbonylmethyl-, 4-Ethylaminocarbonylmethyl-, 4-(1-Methylaminocarbonylethyl)-, 4-(1-Ethylaminocarbonyl-ethyl)-, 4-(2-Methoxy-1-methyl-ethyl)-, 4-(2-Ethoxy-1-methyl-ethyl)-, 4-(2-Methoxy-ethyl)-, 4-(2-Ethoxy-ethyl)- und 4-(2-Propoxy-ethyl)-6-hydrazino-3,4-dihydro-3-oxo-2H-1,4-benzoxazin sowie 4-Methyl-, 4-Ethyl-, 4-Propyl-, 4-Isopropyl-, 4-Butyl-, 4-sec-Butyl-, 4-Isobutyl, 4-Cyanomethyl, 4-(1-Cyano-ethyl)-, 4-(2-Cyano-ethyl)-, 4-Methoxymethyl-, 4-Ethoxymethyl-, 4-Difluormethyl-, 4-Allyl-, 4-(3-Methyl-2-buten-1-yl)-, 4-(2-Buten-1-yl)-, 4-Propargyl-, 4-(2-Butin-1-yl)-, 4-Methoxycarbonylmethyl-, 4-Ethoxycarbonylmethyl-, 4-(1-Methoxycarbonylethyl)-, 4-(1-Ethoxycarbonyl-ethyl)-, 4-Methylaminocarbonylmethyl-, 4-Ethylaminocarbonylmethyl-, 4-(1-Methylaminocarbonyl-ethyl)-, 4-(1-Ethylaminocarbonyl-ethyl)-, 4-(2-Methoxy-1-methyl-ethyl)-, 4-(2-Ethoxy-1-methyl-ethyl)-, 4-(2-Methoxy-ethyl)-, 4-(2-Ethoxy-ethyl)- und 4-(2-Propoxy-ethyl)-7-fluor-6-hydrazino-3,4-dihydro-3-oxo-2H-1,4-benzoxazin.

Die Ausgangsstoffe der Formel (II) - in welcher X für Halogen steht - sind noch nicht aus der Literatur bekannt. Man erhält die neuen Verbindungen der Formel (IIa),

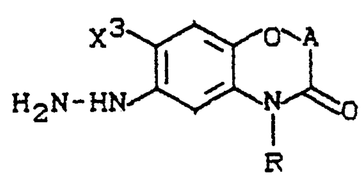

(IIa)

in welcher
$X^3$ für Halogen steht und
A und R die oben angegebenen Bedeutungen haben,
wenn man entsprechende Amino-benzoxazinone der allgemeinen Formel (X)

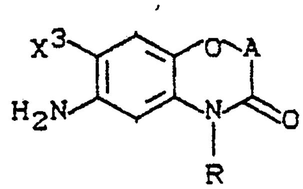

(X)

in welcher
A, R und $X^3$ die oben angegebene Bedeutung haben,
mit Nitriten, wie z. B. Natrium- oder Kalium-nitrit, in Gegenwart von Säuren und/oder Verdünnungsmitteln, wie z. B. Salzsäure, Schwefelsäure und/oder Essigsäure, bei Temperaturen zwischen 0 °C und 30 °C umsetzt, anschließend mit Reduktionsmitteln, wie z. B. Zink, Zinn-(II)-chlorid, Titan-(III)-chlorid oder Natrium-(hydrogen) sulfit, in Gegenwart von Säuren, wie z. B. Salzsäure, bei Temperaturen zwischen 0 °C und 30

°C umsetzt und gegebenenfalls anschließend mit Basen, wie z. B. wäßrigem Ammoniak oder Natronlauge, bei Temperaturen zwischen 0 °C und 30 °C umsetzt und nach üblichen Methoden aufarbeitet.

Verbindungen der Formel (II) in welcher X für Wasserstoff steht sind dagegen bereits bekannt (vgl. DE-OS 27 40 836).

In der Formel (IIa) steht $X^3$ vorzugsweise für Fluor oder Chlor, insbesondere für Fluor und A und R haben vorzugsweise bzw. besonders bevorzugt diejenigen Bedeutungen, die bereits oben bei der Beschreibung der erfindungsgemäßen Verbindungen der Formel (I) vorzugsweise bzw. als insbesondere bevorzugt für A und B angegeben wurden.

Die als Zwischenprodukte zu verwendenden Aminobenzoxazine sind durch die Formel (X) allgemein definiert.

In Formel (X) haben A, R und $X^3$ vorzugsweise bzw. insbesondere diejenigen Bedeutungen, die bereits oben im Zusammenhang mit der Beschreibung der erfindungsgemäßen Verbindungen der Formel (IIa) vorzugsweise bzw. als insbesondere bevorzugt für A, R und $X^3$ angegeben wurden.

Als Beispiele für die Zwischenprodukte der Formel (X) seien genannt:
6-Amino-3,4-dihydro-3-oxo-2H-1,4-benzoxazin, 7-Chlor-und 7-Fluor-6-amino-3,4-dihydro-3-oxo-2H-1,4-benzoxazin, 4-Methyl-, 4-Ethyl-, 4-Propyl-, 4-Isopropyl-, 4-Butyl-, 4-sec-Butyl-, 4-Isobutyl, 4-Cyanomethyl-, 4-(1-Cyano-ethyl)-, 4-(2-Cyano-ethyl)-, 4-Methoxymethyl-, 4-Ethoxymethyl, 4-Difluormethyl-, 4-Allyl-, 4-(3-Methyl-2-buten-1-yl)-, 4-(2-Buten-1-yl)-, 4-Propargyl-, 4-(2-Butin-1-yl)-, 4-Methoxycarbonylmethyl-, 4-Ethoxycarbonylmethyl-, 4-(1-Methoxycarbonylethyl)-, 4-(1-Ethoxycarbonyl-ethyl)-, 4-Methylaminocarbonylmethyl-, 4-Ethylaminocarbonylmethyl-, 4-(1-Methylaminocarbonyl-ethyl)-, 4-(1-Ethylaminocarbonyl-ethyl)-, 4-Methoxy-1-methyl- ethyl)-, 4-(2-Ethoxy-1-methyl-ethyl)-, 4-(2-Methoxy-ethyl)-, 4-(2-Ethoxy-ethyl)- und 4-(2-Propoxy-ethyl)-6-amino-3,4-dihydro-3-oxo-2H-1,4-benzoxazin sowie 4-Methyl-, 4-Ethyl-, 4-Propyl-, 4-Isopropyl-, 4-Butyl-, 4-sec-Butyl-, 4-Isobutyl, 4-Cyanomethyl-, 4-(1-Cyano-ethyl)-, 4-(2-Cyano-ethyl)-, 4-Methoxymethyl, 4-Ethoxymethyl-, 4-Difluormethyl-, 4-Allyl-, 4-(3-Methyl-2-buten-1-yl)-, 4-(2-Buten-1-yl)-, 4-Propargyl-, 4-(2-Butin-1-yl)-, 4-Methoxycarbonylmethyl-, 4-Ethoxycarbonylmethyl-, 4-(1-Methoxycarbonylethyl)-, 4-(1-Ethoxycarbonylethyl)-, 4-Methylaminocarbonylmethyl-, 4-Ethylaminocarbonylmethyl-, 4-(1-Methylaminocarbonyl-ethyl)-, 4-(1-Ethylaminocarbonyl-ethyl)-, 4-(2-Methoxy-1-methyl-ethyl-4-(2-Ethoxy-1-methyl-ethyl)-, 4-(2-Methoxy-ethyl)-, 4-(2-Ethoxy-ethyl)- und 4-(2-Propoxy-ethyl)-7-fluor-6-amino-3,4-dihydro-3-oxo-2H-1,4-benzoxazin.

Die Amino-benzoxazinone der Formel (X) sind bekannt und/oder können nach an sich bekannten Verfahren hergestellt werden (vgl. EP-A 170 191, EP-A 237 899).

Die beim erfindungsgemäßen Verfahren (a) zur Herstellung von Verbindungen der Formel (I) weiter als Ausgangsstoffe zu verwendenden 1,3-Dicarbonylverbindungen sind durch die Formel (III) allgemein definiert.

In Formel (III) haben $R^1$, $R^2$ und $R^3$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$ und $R^3$ angegeben wurden.

Als Beispiele für die Ausgangsstoffe der Formel (III) seien genannt:
Malonaldehyd-tetraethylacetal, 3-Oxo-butyraldehyd-dimethylacetal, Pentan-2,4-dion, 3-Nitro-, 3-Fluor-, 3-Chlor-, 3-Brom-, 3-Methyl-, 3-Ethyl-, 3-Propyl-, 3-Butyl- und 3-Trifluormethyl-pentan-2,4-dion, 1,1,1,-Trifluorpentan-2,4-dion, 1,1,1,5,5,5-hexafluor-pentan-2,4-dion, 2-Formyl- und 2-Acetyl-cyclopentanon sowie 2-Formyl- und 2-Acetyl-cyclohexanon.

Die Ausgangsstoffe der Formel (III) sind bekannte organischen Synthesechemikalien.

Das erfindungsgemäße Verfahren (a) zur Herstellung der neuen substituierten Benzoxazinone der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Alkohole, wie Methanol, Ethanol, Propanol, Isopropanol, Butanol, Isobutanol, sec-Butanol und tert-Butanol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Auch Wasser kann - gegebenenfalls im Gemisch mit den oben angegebenen organischen Lösungsmitteln - als Verdünnungsmittel bei Verfahren (a) verwendet werden.

Die Reaktionstemperaturen können bei dem erfindungsgemäßen Verfahren (a) in einem größeren Bereich variiert werden. Im allgemeinen arbeitet man bei Temperaturen zwischen 0 °C und 150 °C,

vorzugsweise bei Temperaturen zwischen 20 °C und 100 °C. Verfahren (a) wird im allgemeinen bei Normaldruck durchgeführt.

Zur Durchführung des erfindungsgemäßen Verfahrens (a) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel durchgeführt, und das Reaktionsgemisch wird mehrere Stunden bei der jeweils erforderlichen Temperatur gerührt. Die Aufarbeitung erfolgt bei dem erfindungsgemäßen Verfahren (a) jeweils nach üblichen Methoden.

Auf die beim erfindungsgemäßen Verfahren (b) als Ausgangsstoffe einzusetzenden Hydrazino-benzoxazinone der Formel (II) ist bereits im Zusammenhang mit der Beschreibung der Ausgangsstoffe für Verfahren (a) eingegangen worden.

Die beim erfindungsgemäßen Verfahren (b) zur Herstellung von Verbindungen der Formel (I) weiter als Ausgangsstoffe zu verwendenden N-Alkoxycarbonylimidsäureester sind durch die Formel (IV) allgemein definiert.

In Formel (IV) hat $R^4$ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für $R^4$ angegeben wurde und $R^6$ steht vorzugsweise für $C_1$-$C_4$-Alkyl, insbesondere für Methyl oder Ethyl.

Als Beispiele für die Ausgangsstoffe der Formel (IV) seien genannt:

N-Methoxycarbonyl-O-methyl-, N-Ethoxycarbonyl-O-methyl-, N-Methoxycarbonyl-O-ethyl- und N-Ethoxycarbonyl-O-ethyl-acetimidester, -formimidester, -propionimidester, -butyrimidester und -trifluoracetimidester.

Die N-Alkoxycarbonyl-carbonimidsäureester der Formel (IV) sind bekannte organische Synthesechemikalien.

Das erfindungsgemäße Verfahren (b) zur Herstellung der neuen substituierten Benzoxazinone der Formel (I) wird vorzugsweise unter Verwendung von Verdünnungsmitteln durchgeführt. Als Verdünnungsmittel kommen dabei prak tisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ether, Methylisopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Die Reaktionstemperaturen können bei dem erfindungsgemäßen Verfahren (b) in einem größeren Bereich variiert werden. Im allgemeinen arbeitet man bei Temperaturen zwischen 0 °C und 200 °C, vorzugsweise bei Temperaturen zwischen 20 °C und 150 °C.

Zur Durchführung des erfindungsgemäßen Verfahrens (b) setzt man je Mol Hydrazino-benzoxazinon der Formel (II) im allgemeinen zwischen 1 und 2 Mol, vorzugsweise zwischen 1,2 und 1,5 Mol, N-Alkoxycarbonyl-carbonimidsäureester der Formel (IV) ein. Die Reaktionskomponenten werden im allgemeinen bei Raumtemperatur zusammen gegeben und das Reaktionsgemisch wird, gegebenenfalls bei erhöhter Temperatur, bis zum Reaktionsende gerührt. Die Aufarbeitung kann nach üblichen Methoden durchgeführt werden.

Die beim erfindungsgemäßen Verfahren (c) zur Herstellung von Verbindungen der Formel (I) als Ausgangsstoffe zu verwendenden Acylhydrazino-benzoxazinone sind durch die Formel (V) allgemein definiert.

In Formel (V) haben A, R, $R^4$ und X vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, R, $R^4$ und X angegeben wurden.

Beispiele für die Ausgangsstoffe der Formel (V) sind in der nachstehenden Tabelle 2 aufgeführt:

Tabelle 2 : Beispiele für die Ausgangsstoffe der Formel (V)

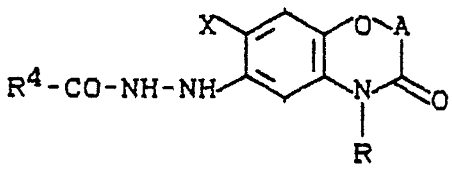

(V)

| A | R | $R^4$ | X |
|---|---|---|---|
| $CH_2$ | H | H | H |
| $CH_2$ | H | $CH_3$ | H |
| $CH_2$ | H | H | F |
| $CH_2$ | H | H | Cl |
| $CH_2$ | $CH_3$ | H | H |
| $CH_2$ | $CH_3$ | $CH_3$ | H |
| $CH_2$ | H | $C_2H_5$ | F |
| $CH_2$ | H | $C_3H_7$ | F |
| $CH_2$ | H | $C_4H_9$ | F |
| $CH_2$ | H | $-CH(CH_3)_2$ | F |
| $CH_2$ | H | $-CH_2CH(CH_3)_2$ | F |
| $CH_2$ | H | $-C(CH_3)_3$ | F |
| $CH_2$ | $CH_3$ | H | F |
| $CH_2$ | $CH_3$ | $CH_3$ | F |
| $CH_2$ | $C_2H_5$ | $CH_3$ | F |
| $CH_2$ | $C_2H_5$ | $C_2H_5$ | F |
| $CH_2$ | $C_3H_7$ | $CH_3$ | F |
| $CH_2$ | $C_4H_9$ | $CH_3$ | F |

Tabelle 2 - Fortsetzung

| A | R | $R^4$ | X |
|---|---|---|---|
| $CH_2$ | $-CH_2CN$ | $CH_3$ | F |
| $CH_2$ | $-CH_2OCH_3$ | $CH_3$ | F |
| $CH_2$ | $-CH_2CH{=}CH_2$ | $CH_3$ | F |
| $CH_2$ | $-CH_2C{\equiv}CH$ | $CH_3$ | F |
| $CH_2$ | $-CH_2C{\equiv}CH$ | $CH_3$ | H |
| $CH_2$ | $-CH_2C{\equiv}CH$ | $C_2H_5$ | F |
| $CH_2$ | $-CH_2C{\equiv}CH$ | $C_3H_7$ | F |
| $CH_2$ | $-CH_2C{\equiv}CH$ | $C_4H_9$ | F |
| $CH_2$ | $-CH_2$ 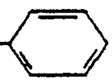 | $CH_3$ | F |
| $CH_2$ | $-CH_2C{\equiv}N$ | $-C(CH_3)_3$ | F |
| $CH_2$ | $-CH_2C{\equiv}N$ | $CH_3$ | H |
| $CH_2$ | $-CH_2CH{=}CH_2$ | $CH_3$ | H |
| $CH_2$ | $-CH_2CH_2C{\equiv}N$ | $CH_3$ | F |
| $CH_2$ | H | $-C(CH_3)_3$ | H |
| $CH_2$ | $-CH_2C{\equiv}CH$ | $-C(CH_3)_3$ | H |
| $CH_2$ | $-CH_2C{\equiv}CH$ | $-C(CH_3)_3$ | F |
| $CH_2$ | $-CHF_2$ | $CH_3$ | F |
| $CH_2$ | $-CH_2C{\equiv}CH$ | $CF_3$ | H |
| $CH_2$ | $-CH_2C{\equiv}CH$ | $CF_3$ | F |
| $CH_2$ | $-CH_2C{\equiv}CH$ | $-CHCl_2$ | H |
| $CH_2$ | $-CH_2C{\equiv}CH$ | $-CHCl_2$ | F |

Tabelle 2 - Fortsetzung

| A | R | $R^4$ | X |
|---|---|---|---|
| $CHCH_3$ | H | H | H |
| $CHCH_3$ | H | $CH_3$ | H |
| $CHCH_3$ | H | H | F |
| $CHCH_3$ | H | H | Cl |
| $CHCH_3$ | $CH_3$ | H | H |
| $CHCH_3$ | $CH_3$ | $CH_3$ | H |
| $CHCH_3$ | H | $C_2H_5$ | F |
| $CHCH_3$ | H | $C_3H_7$ | F |
| $CHCH_3$ | H | $C_4H_9$ | F |
| $CHCH_3$ | H | $-CH(CH_3)_2$ | F |
| $CHCH_3$ | H | $-CH_2CH(CH_3)_2$ | F |
| $CHCH_3$ | H | $-C(CH_3)_3$ | F |
| $CHCH_3$ | $CH_3$ | H | F |
| $CHCH_3$ | $CH_3$ | $CH_3$ | F |
| $CHCH_3$ | $C_2H_5$ | $CH_3$ | F |
| $CHCH_3$ | $C_2H_5$ | $C_2H_5$ | F |
| $CHCH_3$ | $C_3H_7$ | $CH_3$ | F |
| $CHCH_3$ | $C_4H_9$ | $CH_3$ | F |
| $CHCH_3$ | $-CH_2CN$ | $CH_3$ | F |
| $CHCH_3$ | $-CH_2OCH_3$ | $CH_3$ | F |
| $CHCH_3$ | $-CH_2CH=CH_2$ | $CH_3$ | F |

Tabelle 2 - Fortsetzung

| A | R | $R^4$ | X |
|---|---|---|---|
| $CHCH_3$ | $-CH_2C\equiv CH$ | $CH_3$ | F |
| $CHCH_3$ | $-CH_2C\equiv CH$ | $CH_3$ | H |
| $CHCH_3$ | $-CH_2C\equiv CH$ | $C_2H_5$ | F |
| $CHCH_3$ | $-CH_2C\equiv CH$ | $C_3H_7$ | F |
| $CHCH_3$ | $-CH_2C\equiv CH$ | $C_4H_9$ | F |
| $CHCH_3$ | $-CH_2$ 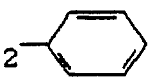 | $CH_3$ | F |
| $CHCH_3$ | $-CH_2C\equiv N$ | $-C(CH_3)_3$ | F |
| $CHCH_3$ | $-CH_2C\equiv N$ | $CH_3$ | H |
| $CHCH_3$ | $-CH_2CH=CH_2$ | $CH_3$ | H |
| $CHCH_3$ | $-CH_2CH_2C\equiv N$ | $CH_3$ | F |
| $CHCH_3$ | H | $-C(CH_3)_3$ | H |
| $CHCH_3$ | $-CH_2C\equiv CH$ | $-C(CH_3)_3$ | H |
| $CHCH_3$ | $-CH_2C\equiv CH$ | $-C(CH_3)_3$ | F |
| $CHCH_3$ | $-CHF_2$ | $CH_3$ | F |
| $CHCH_3$ | $-CH_2C\equiv CH$ | $CF_3$ | H |
| $CHCH_3$ | $-CH_2C\equiv CH$ | $CF_3$ | F |
| $CHCH_3$ | $-CH_2C\equiv CH$ | $-CHCl_2$ | H |
| $CHCH_3$ | $-CH_2C\equiv CH$ | $-CHCl_2$ | F |

Die Ausgangsstoffe der Formel (V) sind noch nicht aus der Literatur bekannt. Man erhält die neuen Verbindungen der Formel (V), wenn man entsprechende Hydrazino-benzoxazinone der Formel (II)

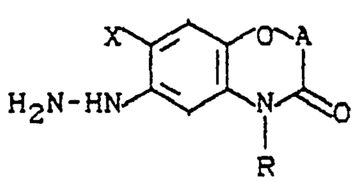

(II)

in welcher
A, R und X die oben angegebenen Bedeutungen haben,
mit Acylierungsmitteln der allgemeinen Formel (XI)

$R^4$ - CO - $X^4$ (XI)
in welcher
$R^4$ die oben angegebene Bedeutung hat und
$X^4$ für Halogen steht,
gegebenenfalls in Gegenwart von Säureakzeptoren, wie z. B. Triethylamin oder Pyridin, und gegebenenfalls in Gegenwart von Verdünnungsmitteln, wie z. B. Toluol oder Methylenchlorid, bei Temperaturen zwischen -20 °C und +50 °C umsetzt und nach üblichen Methoden aufarbeitet.

Auf die als Zwischenprodukte zu verwendenden Hydrazino-benzoxazinone der Formel (II) ist bereits im Zusammen hang mit der Beschreibung der Ausgangsstoffe für Verfahren (a) eingegangen worden.

Die weiter als Zwischenprodukte zu verwendenden Acylierungsmittel sind durch die Formel (XI) allgemein definiert.

In Formel (XI) hat $R^4$ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für $R^4$ angegeben wurde und $X^4$ steht vorzugsweise für Chlor.

Als Beispiele für die Ausgangsstoffe der Formel (XI) seien genannt:
Acetylchlorid, Propionylchlorid, Buttersäurechlorid, Isobuttersäurechlorid, Pivaloylchlorid und Dichloracetylchlorid.

Die Zwischenprodukte der Formel (XI) sind bekannte organische Synthesechemikalien.

Das bei Verfahren (c) weiter als Ausgangsstoff zu verwendende Phosgen ist ebenfalls eine bekannte Synthesechemikalie.

Das erfindungsgemäße Verfahren (c) wird vorzugsweise unter Verwendung von Verdünnungsmitteln durchgeführt. Es kommen vor allem diejenigen organischen Lösungsmittel in Betracht, die bereits oben für Verfahren (b) angegeben wurden.

Als Säureakzeptoren können bei dem erfindungsgemäßen Verfahren (c) alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (c) in einem größeren Bereich variiert werden. Im allgemeinen arbeitet man bei Temperaturen zwischen 0 °C und 150 °C, vorzugsweise bei Temperaturen zwischen 10 °C und 120 °C.

Zur Durchführung des erfindungsgemäßen Verfahrens (c) setzt man je Mol Acylhydrazino-benzoxazinon der Formel (V) im allgemeinen zwischen 1 und 2 Mol, vorzugsweise zwischen 1,1 und 1,5 Mol, Phosgen ein. Die Reaktionskomponenten werden im allgemeinen bei Raumtemperatur oder unter leichtem Kühlen zusammengegeben und das Reaktionsgemisch wird, gegebenenfalls bei erhöhter Temperatur bis zum Reaktionsende gerührt. Die Aufarbeitung kann nach üblichen Methoden durchgeführt werden.

Die beim erfindungsgemäßen Verfahren (d) zur Herstellung von Verbindungen der Formel (I) als Ausgangsstoffe zu verwendenden Verbindungen sind durch die Formel (VI) allgemein definiert.

In Formel (VI) haben A, X und Y vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, X und Y angegeben wurden, und Q steht vorzugsweise für Cyano, Carboxy, Methoxycarbonyl oder Ethoxycarbonyl.

Beispiele für die Ausgangsstoffe der Formel (VI) sind in der nachstehenden Tabelle 3 aufgeführt:

<u>Tabelle 3</u>: Beispiele für die Ausgangsstoffe der Formel (VI)

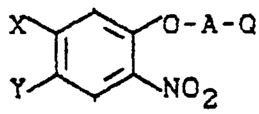

(VI)

| A | Q | X | Y |
|---|---|---|---|
| $CH_2$ | CN | H | 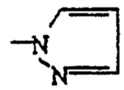 |
| $CH_2$ | CN | F | 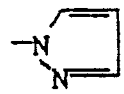 |

Tabelle 3 - Fortsetzung

| A | Q | X | Y |
|---|---|---|---|
| $CH_2$ | $COOCH_3$ | F | 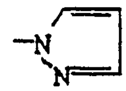 |
| $CH_2$ | $COOC_2H_5$ | F | 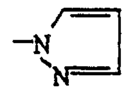 |
| $CH_2$ | $COOCH_3$ | F | 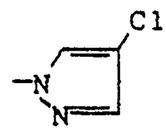 |
| $CH_2$ | $COOCH_3$ | F | 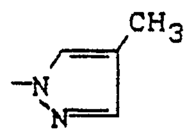 |
| $CH_2$ | $COOCH_3$ | F | 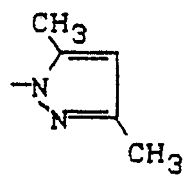 |
| $CH_2$ | $COOC_2H_5$ | F | 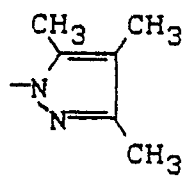 |
| $CH_2$ | $COOCH_3$ | H | 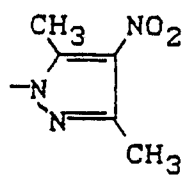 |
| $CH_2$ | $COOC_2H_5$ | F | 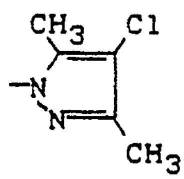 |

Tabelle 3 - Fortsetzung

| A | Q | X | Y |
|---|---|---|---|
| $CH_2$ | CN | F | 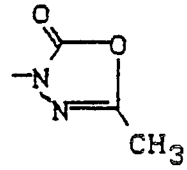 |
| $CH_2$ | $COOCH_3$ | F | 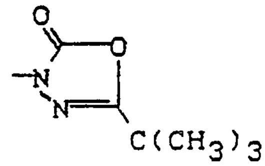 |
| $CH_2$ | $COOC_2H_5$ | F | 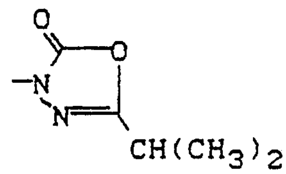 |
| $CH_2$ | CN | H | 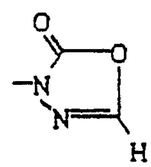 |
| $CH_2$ | $COOCH_3$ | H | 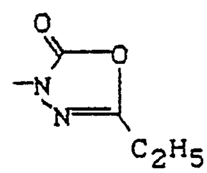 |
| $CH_2$ | CN | H | 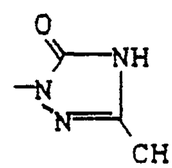 |
| $CH_2$ | $COOCH_3$ | F | 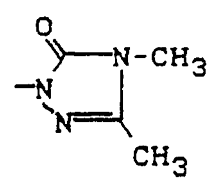 |

Tabelle 3 - Fortsetzung

| A | Q | X | Y |
|---|---|---|---|
| $CH_2$ | $COOC_2H_5$ | F | 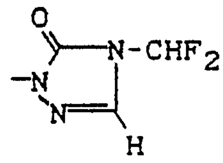 |
| $CH_2$ | CN | H | 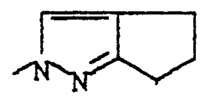 |
| $CH_2$ | CN | F | 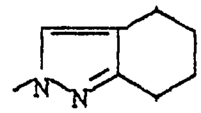 |
| $CH_2$ | $COOCH_3$ | F | 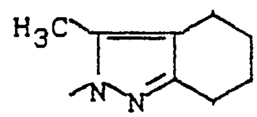 |
| $CH_2$ | $COOC_2H_5$ | F | 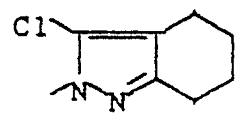 |
| $CH_2$ | $COOCH_3$ | F | 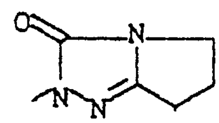 |
| $CH_2$ | $COOC_2H_5$ | F | 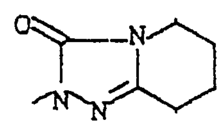 |
| $CH_2$ | $COOC_2H_5$ | F | 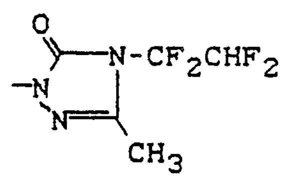 |
| $CH_2$ | CN | F | 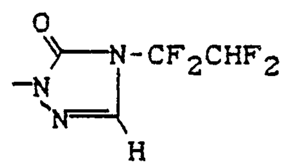 |

<u>Tabelle 3</u> - Fortsetzung

| A | Q | X | Y |
|---|---|---|---|
| $CH_2$ | $COOCH_3$ | F | 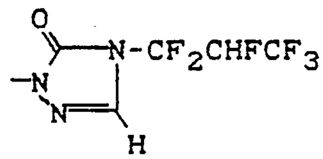 |
| $CH_2$ | COOH | F | 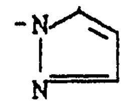 |
| $CH_2$ | COOH | F | 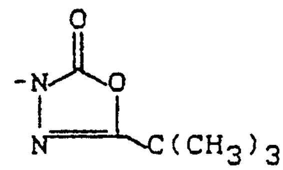 |
| $CHCH_3$ | CN | H | 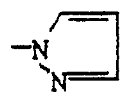 |
| $CHCH_3$ | CN | F | 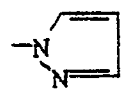 |
| $CHCH_3$ | $COOCH_3$ | F | 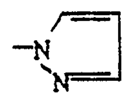 |
| $CHCH_3$ | $COOC_2H_5$ | F | 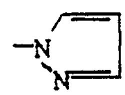 |
| $CHCH_3$ | $COOCH_3$ | F | 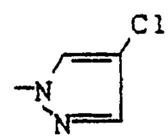 |
| $CHCH_3$ | $COOCH_3$ | F | 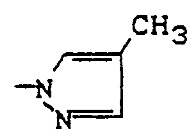 |

Tabelle 3 - Fortsetzung

| A | Q | X | Y |
|---|---|---|---|
| $CHCH_3$ | $COOCH_3$ | F | 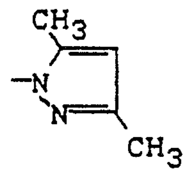 |
| $CHCH_3$ | $COOC_2H_5$ | F | 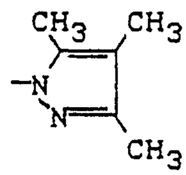 |
| $CHCH_3$ | $COOCH_3$ | H | 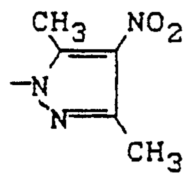 |
| $CHCH_3$ | $COOC_2H_5$ | F | 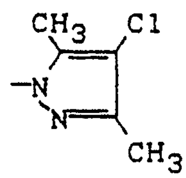 |
| $CHCH_3$ | CN | F | 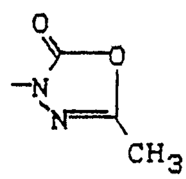 |
| $CHCH_3$ | $COOCH_3$ | F | 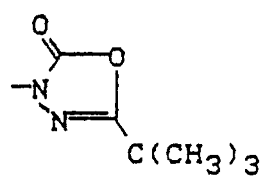 |
| $CHCH_3$ | $COOC_2H_5$ | F | 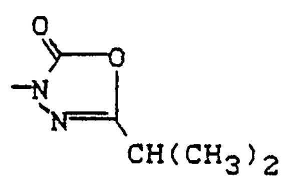 |

<u>Tabelle 3</u> - Fortsetzung

| A | Q | X | Y |
|---|---|---|---|
| $CHCH_3$ | CN | H | 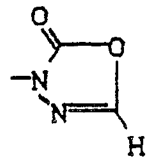 |
| $CHCH_3$ | $COOCH_3$ | H | 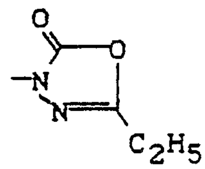 |
| $CHCH_3$ | CN | H | 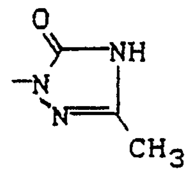 |
| $CHCH_3$ | $COOCH_3$ | F | 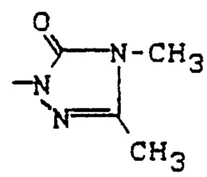 |
| $CHCH_3$ | $COOC_2H_5$ | F | 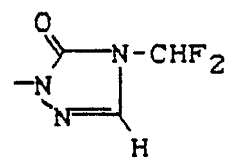 |
| $CHCH_3$ | CN | H | 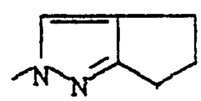 |
| $CHCH_3$ | CN | F | 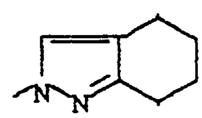 |
| $CHCH_3$ | $COOCH_3$ | F | 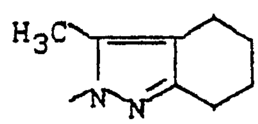 |

Tabelle 3 - Fortsetzung

| A | Q | X | Y |
|---|---|---|---|
| $CHCH_3$ | $COOC_2H_5$ | F | 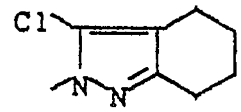 |
| $CHCH_3$ | $COOCH_3$ | F | 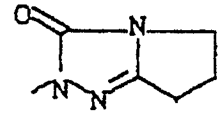 |
| $CHCH_3$ | $COOC_2H_5$ | F | 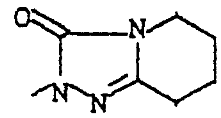 |
| $CHCH_3$ | $COOC_2H_5$ | F | 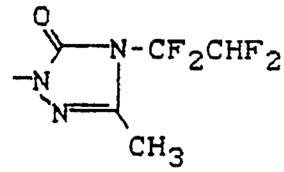 |
| $CHCH_3$ | CN | F | 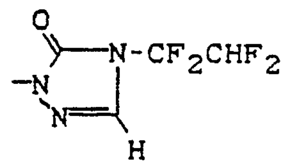 |
| $CHCH_3$ | $COOCH_3$ | F | 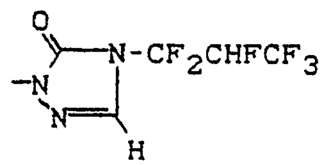 |
| $CHCH_3$ | COOH | F | 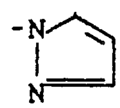 |
| $CHCH_3$ | COOH | F | 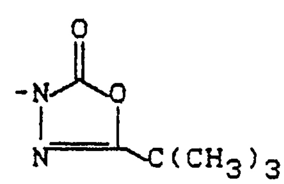 |

Die Ausgangsstoffe der Formel (VI) sind noch nicht aus der Literatur bekannt. Man erhält die neuen Zwischenprodukte der Formel (VI), wenn man Verbindungen der allgemeinen Formel (XII)

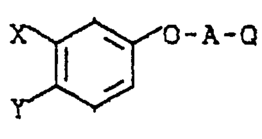

(XII)

in welcher
A, Q, X und Y die oben angegebenen Bedeutungen haben,
mit Nitrierungsmitteln, wie z. B. Salpetersäure, gegebenenfalls in Gegenwart von Schwefelsäure, bei

Temperaturen zwischen -20 °C und +50 °C umsetzt und nach üblichen Methoden aufarbeitet.

Die - ebenfalls neuen - Zwischenprodukte der Formel (XII) können, ausgehend von entsprechenden Arylhydrazinen der allgemeinen Formel (XIII)

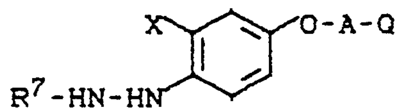

(XIII)

in welcher

A, Q und X die oben angegebenen Bedeutungen haben und

$R^7$ für Wasserstoff bzw. für die Gruppierung $-CO-R^4$ steht - worin $R^4$ die oben angegebene Bedeutung hat, analog zu den erfindungsgemäßen Verfahren (a), (b) oder (c) hergestellt werden.

Die Arylhydrazine der Formel (XIII) sind bekannt und/oder können nach an sich bekannten Verfahren hergestellt werden.

Man erhält die Zwischenprodukte der Formel (XII) auch, wenn man Phenolderivate der Formel (XIV)

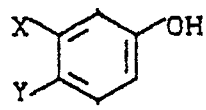

(XIV)

in welcher

X und Y die oben angegebenen Bedeutungen haben,

mit Alkylierungsmitteln der Formel (XV)

$X^5 - A - Q$ (XV),

in welcher

A und Q die oben angegebenen Bedeutungen haben und

$X^5$ für Halogen steht,

in Gegenwart eines Säureakzeptors, wie z. B. Kaliumcarbonat, und in Gegenwart eines Verdünnungsmittels, wie z. B. Aceton oder Acetonitril, bei Temperaturen zwischen 0 °C und 100 °C umsetzt.

Die Phenolderivate der Formel (XIV) können aus entsprechenden Hydroxyarylhydrazinen der Formel (XVI)

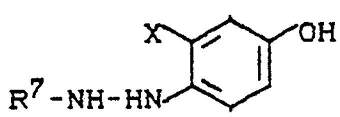

(XVI)

in welcher

$R^7$ und X die oben angegebenen Bedeutungen haben,

analog zu den erfindungsgemäßen Verfahren (a), (b) oder (c) hergestellt werden.

Die Alkylierungsmittel der Formel (XV) und die Hydroxyarylhydrazine der Formel (XVI) sind bekannt und/oder können nach an sich bekannten Verfahren hergestellt werden.

Man erhält die Phenolderivate der Formel (XIV) ferner auch, wenn man entsprechende Methylether der Formel (XVII)

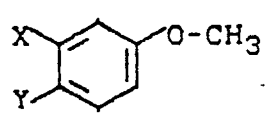

(XVII)

in welcher

X und Y die oben angegebenen Bedeutungen haben,

nach üblichen Etherspaltungsmethoden, beispielsweise durch Umsetzung mit 48 %iger Bromwasserstoffsäure, bei Temperaturen zwischen 0 °C und 150 °C spaltet.

Die Methylether der Formel (XVII) können aus entsprechenden Methoxyarylhydrazinen der Formel

(XVIII)

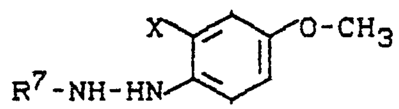

(XVIII)

in welcher
$R^7$ und X die oben angegebenen Bedeutungen haben,
analog zu den erfindungsgemäßen Verfahren (a), (b) oder (c) hergestellt werden.

An den Zwischenprodukten der Formeln (XIV) und (XVII) können gegebenenfalls auch Reaktionen analog zu den erfindungsgemäßen Verfahren (f) und (h) - zu entspechenden Veränderungen des Substituenten Y - durchgeführt werden.

Bei der Umsetzung von Verbindungen der Formel (XIV) mit Nitrierungsmitteln kann gegebenenfalls auch eine zweifache Nitrierung, beispielsweise zu Verbindungen der Formel (XIX)

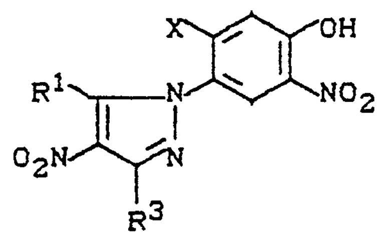

(XIX)

in welcher
$R^1$, $R^3$ und X die oben angegebenen Bedeutungen haben,
erreicht werden. Die so erhaltenen Verbindungen der Formel (XIX) können mit Alkylierungsmitteln der Formel (XV) - oben, wie dort beschrieben - weiter zu entsprechenden Ausgangsstoffen der Formel (VI) umgesetzt werden.

Das erfindungsgemäße Verfahren (d) zur Herstellung der neuen Verbindungen der Formel (I) wird unter Einsatz eines Reduktionsmittels durchgeführt. Als Reduktionsmittel für Verfahren (d) sind vor allem Wasserstoff in Gegegenwart eines Katalysators oder Eisen in Kombination mit einer Säure, wie z. B. Salzsäure, geeignet.

Das erfindungsgemäße Verfahren (d) wird gegebenenfalls unter Verwendung eines Katalysators durchgeführt. Als Beispiele hierfür seien Raney-Nickel, Platin und Palladium genannt. Vorzugsweise wird Raney-Nickel für Verfahren (d) eingesetzt.

Verfahren (d) wird in Gegenwart eines Verdünnungsmittels durchgeführt. Vorzugsweise werden Alkohole, wie Methanol, Ethanol, Propanol oder Isopropanol, Ether, wie Glycoldimethylether, Diglycoldimethylether, Tetrahydrofuran oder Dioxan, oder Ester, wie Essigsäuremethylester oder Essigsäureethylester, daneben gegebenenfalls auch Wasser als Lösungsmittel für Verfahren (d) eingesetzt.

Die Reaktionstemperaturen können bei dem erfindungsgemäßen Verfahren (d) in einem größeren Bereich variiert werden. Im allgemeinen arbeitet man bei Temperaturen zwischen 0 °C und 150 °C, vorzugsweise bei Temperaturen zwischen 20 °C und 100 °C.

Verfahren (d) wird im allgemeinen bei Normaldruck oder erhöhtem Druck bis etwa 200 bar, vorzugsweise bis etwa 100 bar, durchgeführt.

Verfahren (d) kann unter den bei katalytischen Hydrierungen üblichen Bedingungen durchgeführt werden. In einer bevorzugten Ausführungsform von Verfahren (d) wird die Ausgangsverbindung der Formel (VI) mit dem Verdünnungsmittel und dem Katalysator vermischt und dann so lange Wasserstoff zudosiert, bis kein Verbrauch von Wasserstoff mehr festzustellen ist. Nach dem Ende der Hydrierung filtriert man das Reaktionsgemisch und erhält nach Einengen des Filtrates das Rohprodukt als Rückstand, welches auf übliche Weise, beispielsweise durch Säulenchromatographie, gereinigt werden kann.

In einer anderen bevorzugten Ausführungsform von Verfahren (d) wird Eisen mit einer Säure, vorzugsweise Salzsäure, in einem geeigneten Lösungsmittel vorgelegt, die Ausgangsverbindungen der Formel (VI) wird, gegebenenfalls bei erhöhter Temperatur, langsam dazu gegeben und das Reaktionsgemisch wird bis zum Ende der Umsetzung gerührt. Die Aufarbeitung erfolgt nach üblichen Methoden.

Die beim erfindungsgemäßen Verfahren (e) zur Herstellung von Verbindungen der Formel (I) als

Ausgangsstoffe zu verwendenden Benzoxazinone sind durch die Formel (Ia) allgemein definiert.

In Formel (Ia) haben A, X und Y vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, X und Y angegeben wurden.

Die Ausgangsstoffe der Formel (Ia) für Verfahren (e) sind erfindungsgemäße, neue Verbindungen; sie können nach den erfindungsgemäßen Verfahren (a), (b), (c) oder (d) hergestellt werden.

Die beim erfindungsgemäßen Verfahren (e) zur Herstellung von Verbindungen der Formel (I) weiter als Ausgangsstoffe zu verwendenden Alkylierungsmittel sind durch die Formel (VII) allgemein definiert.

In Formel (VII) hat R vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für R angegeben wurde und $X^1$ steht vorzugsweise für Chlor, Brom oder Iod.

Als Beispiele für die Ausgangsstoffe der Formel (VII) seien genannt:

Methyl-, Ethyl-, Propyl-, Isopropyl-, Butyl-, Isobutyl-, sec-Butyl-, tert-Butyl-, Allyl-, Propargyl-, Benzyl-, Picolyl-, Methoxymethyl-, Ethoxymethyl-, Cyanomethyl-und Cyanoethyl-chlorid sowie die entsprechenden Bromide und Iodide, Chlor- und Bromessigsäure-methylester und -ethylester sowie α-Chlor- und α-Brom-propionsäure-methylester und -ethylester.

Die Ausgangsstoffe der Formel (VII) sind bekannte organische Synthesechemikalien.

Das erfindungsgemäße Verfahren (e) wird vorzugsweise unter Verwendung von Verdünnungsmitteln durchgeführt. Es kommen vor allem diejenigen organischen Lösungsmittel in Betracht, die bereits oben für Verfahren (b) angegeben wurden.

Das erfindungsgemäße Verfahren (e) wird vorzugsweise in Gegenwart eines Säureakzeptors durchgeführt. Es kommen vor allem diejenigen Säureakzeptoren in Betracht, die bereits oben für Verfahren (c) angegeben wurden, ferner jedoch auch Alkalimetallhydride, wie z. B. Natrium- und Kalium-hydrid.

Die Reaktionstemperaturen können bei dem erfindungsgemäßen Verfahren (e) in einem größeren Bereich variiert werden. Im allgemeinen arbeitet man bei Temperaturen zwischen -20 °C und +200 °C, vorzugsweise bei Temperaturen zwischen 0 °C und 150 °C.

Das erfindungsgemäße Verfahren (e) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (e) werden die jeweils benötigten Ausgangsstoffe im allgemeinen in angenähert äquimolaren Mengen eingesetzt. Es ist jedoch auch möglich, eine der beiden jeweils einge setzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (e) jeweils nach üblichen Methoden.

Die beim erfindungsgemäßen Verfahren (f) zur Herstellung von Verbindungen der Formel (I) als Ausgangsstoffe zu verwendenden Benzoxazinone sind durch die Formel (Ib) allgemein definiert.

In Formel (Ib) haben A, R, $R^4$ und X vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, R, $R^4$ und X angegeben wurden.

Die Ausgangsstoffe der Formel (Ib) für Verfahren (f) sind erfindungsgemäße, neue Verbindungen; sie können nach dem erfindungsgemäßen Verfahren (b) hergestellt werden.

Die beim erfindungsgemäßen Verfahren (f) weiter als Ausgangsstoffe zu verwendenden Alkylierungsmittel sind durch die Formel (VIII) allgemein definiert. In Fomel (VIII) steht vorzugsweise

$R^5$ für jeweils gegebenenfalls durch Fluor und/oder Chlor substituiertes $C_1$-$C_4$-Alkyl, $C_3$-$C_4$-Alkenyl oder $C_3$-$C_4$-Alkinyl und

$X^2$ für Chlor, Brom oder Iod.

Als Beispiele für die Ausgangsstoffe der Formel (VIII) seien genannt:

Methyl-, Ethyl-, Propyl-, Isopropyl-, Butyl-, Isobutyl-, sec-Butyl-, tert-Butyl-, Allyl- und Propargyl-bromid sowie die entsprechenden Chloride und Iodide.

Die Ausgangsstoffe der Formel (VIII) sind bekannte organische Synthesechemikalien.

Das erfindungsgemäße Verfahren (f) wird vorzugsweise unter Verwendung von Verdünnungsmitteln durchgeführt. Es kommen vor allem diejenigen organischen Lösungsmittel in Betracht, die bereits oben für Verfahren (b) angegeben wurden.

Das erfindungsgemäße Verfahren (f) wird vorzugsweise in Gegenwart eines Säureakzeptors durchgeführt. Es kommen vor allem diejenigen Säureakzeptoren in Betracht, die bereits oben für Verfahren (c) angegeben wurden, ferner jedoch auch Alkalimetallhydride, wie z. B. Natrium- und Kalium-hydrid.

Die Reaktionstemperaturen können bei dem erfindungsgemäßen Verfahren (f) in einem größeren

Bereich variiert werden. Im allgemeinen arbeitet man bei Temperaturen zwischen -20 °C und +200 °C, vorzugsweise bei Temperaturen zwischen 0 °C und 150 °C.

Das erfindungsgemäße Verfahren (f) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (f) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (f) jeweils nach üblichen Methoden.

Die beim erfindungsgemäßen Verfahren (g) zur Herstellung von Verbindungen der Formel (I) als Ausgangsstoffe zu verwendenden Benzoxazinone sind durch die Formel (IX) allgemein definiert.

In Formel (IX) haben A, R, $R^2$, $R^3$ und X vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, R, $R^2$, $R^3$ und X angegeben wurden.

Beispiele für die Ausgangsstoffe der Formel (IX) sind in der nachstehenden Tabelle 4 aufgeführt.

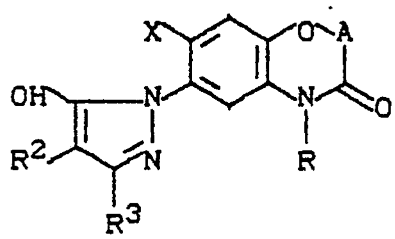

(IX)

<u>Tabelle 4</u>: Beispiele für die Ausgangsstoffe der Formel (IX)

| A | R | $R^2$ | $R^3$ | X |
|---|---|---|---|---|
| $CH_2$ | H | H | $CH_3$ | H |
| $CH_2$ | H | H | $CH_3$ | F |
| $CH_2$ | $CH_3$ | H | $CH_3$ | H |
| $CH_2$ | $CH_3$ | H | $CH_3$ | F |
| $CH_2$ | $CH_3$ | Cl | $CH_3$ | H |
| $CH_2$ | $CH_3$ | Cl | $CH_3$ | F |
| $CH_2$ | $CH_3$ | H | $CF_3$ | H |
| $CH_2$ | $CH_3$ | H | $CF_3$ | F |
| $CH_2$ | $CH_3$ | CN | $CH_3$ | H |
| $CH_2$ | $CH_3$ | CN | $CH_3$ | F |
| $CH_2$ | $C_2H_5$ | H | $CH_3$ | H |
| $CH_2$ | $C_2H_5$ | H | $CH_3$ | F |
| $CH_2$ | $C_3H_7$ | H | $CH_3$ | H |
| $CH_2$ | $C_3H_7$ | H | $CH_3$ | F |
| $CH_2$ | $C_4H_9$ | H | $CH_3$ | H |
| $CH_3$ | $C_4H_9$ | H | $CH_3$ | F |

Die Ausgangsstoffe der Formel (IX) sind noch nicht aus der Literatur bekannt. Man erhält die neuen Verbindungen der Formel (IX), wenn man Hydrazino-benzoxazinone der allgemeinen Formel (II) - oben - mit $\beta$-Ketoestern der allgemeinen Formel (XX)

$$R^3\text{-CO-}\underset{\displaystyle R^2}{\underset{|}{CH}}\text{-COOR}^8 \qquad (XX)$$

in welcher
$R^2$ und $R^3$ die oben angegebenen Bedeutungen haben und
$R^8$ für Alkyl steht,
gegebenenfalls in Gegenwart von Verdünnungsmitteln, wie z. B. Ethanol und/oder Wasser, bei Temperaturen zwischen 0 °C und 150 °C umsetzt.

Die als Zwischenprodukte zu verwendenden $\beta$-Ketoester sind durch die Formel (XX) allgemejn definiert. In Formel (XX) haben $R^2$ und $R^3$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesonders bevorzugt für $R^2$ und $R^3$ angegeben wurden und $R^8$ steht vorzugsweise für Methyl oder Ethyl.

Als Beispiele für die Zwischenprodukte der Formel (XX) seien genannt: Acetessigsäure-methylester und -ethylester, α-Fluor-, α-Chlor-, α-Cyano- und α-Methyl-acetessigsäure-methylester und -ethylester sowie ω,ω,ω-Trifluoracetessigsäure-methylester und -ethylester.

Die Verbindungen der Formel (XX) sind bekannte organische Synthesechemikalien.

Das erfindungsgemäße Verfahren (g) wird unter Verwendung eines Halogenierungsmittels durchgeführt. Es können die üblichen Mittel für die Umwandlung aktivierter Hydroxyverbindungen in davon abgeleitete Halogenverbindungen eingesetzt werden. Als Beispiele für geeignete Halogenierungsmittel seien genannt: Phosgen, Diphosgen, Thionylchlorid, Phosphorylchlorid, Phosphor(III)-chlorid und Phosphor(III)-bromid.

Verfahren (g) wird gegebenenfalls in Gegenwart eines Katalystors durchgeführt. Es können die für die Umwandlung von Hydroxyverbindungen in entsprechende Halogenverbindungen üblichen Katalysatoren, wie z. B. Pyridin, Dimethylformamid, N,N-Diethylanilin, Triphenylphosphin und Triphenylphosphinoxid, verwendet werden.

Verfahren (g) wird gegebenenfalls in Gegenwart eines Verdünnungsmittels durchgeführt. Vorzugsweise kommen inerte organische Lösungsmittel aus der Reihe der halogenierten Kohlenwasserstoffe, wie z. B. Methylenchlorid, Chloroform, Tetrachlormethan oder 1,2-Dichlorethan, in Betracht.

Die Reaktionstemperaturen können bei dem erfindungsge mäßen Verfahren (g) in einem größeren Bereich variiert werden. Im allgemeinen arbeitet man bei Temperaturen zwischen 0 °C und 100 °C, vorzugsweise bei Temperaturen zwischen 10 °C und 90 °C.

Verfahren (g) wird im allgemeinen bei Normaldruck durchgeführt.

Zur Durchführung von Verfahren (g) werden je Mol Ausgangsverbindung der Formel (IX) im allgemeinen zwischen 1 und 100 Mol, vorzugsweise zwischen 2 und 50 Mol, Halogenierungsmittel eingesetzt. Die Reaktionskomponenten werden im allgemeinen bei Raumtemperatur zusammengegeben und das Reaktionsgemisch wird, gegebenenfalls bei erhöhter Temperatur bis zum Ende der Umsetzung gerührt. Das nach Abdestillieren der flüchtigen Komponenten unter vermindertem Druck verbleibende Reaktionsprodukt kann durch Umkristallisation gereinigt werden.

Die beim erfindungsgemäßen Verfahren (h) zur Herstellung von Verbindungen der Formel (I) als Ausgangsstoffe zu verwendenden Benzoxazinone sind durch die Formel (Ic) allgemein definiert.

In Formel (Ic) haben A, R, $R^1$, $R^3$ und X vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, R, $R^1$, $R^3$und X angegeben wurden.

Die Ausgangsstoffe der Formel (Ic) für Verfahren (h) sind erfindungsgemäße, neue Verbindungen; sie können nach dem erfindungsgemäßen Verfahren (a) hergestellt werden.

Verfahren (h) kann nach üblichen Halogenierungs- bzw. Nitrierungsmethoden durchgeführt werden.

Zur Halogenierung werden vorzugsweise elementare Halogene, wie Chlor, Brom oder Iod, oder Halogenverbindungen, wie Sulfurylchlorid oder Natriumhypochlorit, eingesetzt.

Zur Nitrierung wird Salpetersäure, gegebenenfalls in Gegenwart von Schwefelsäure und/oder in Gegenwart eines Nitrits, wie Natriumnitrit, eingesetzt.

Verfahren (h) wird vorzugsweise in Gegenwart eines Verdünnungsmittels durchgeführt. Vorzugsweise kommen inerte organische Lösungsmittel aus der Reihe der halogenierten Kohlenwasserstoffe, wie Methylenchlorid, Chloroform, Tetrachlormethan oder 1,2-Dichlorethan, in Betracht.

Die Reaktionstemperaturen können bei dem erfindungsgemäßen Verfahren (h) in einem größeren Bereich variiert werden. Im allgemeinen arbeitet man bei Temperaturen zwischen -20 °C und +80 °C, vorzugsweise bei Temperaturen zwischen 0 °C und 50 °C.

Das erfindungsgemäße Verfahren (h) wird im allgemeinen unter Normaldruck durchgeführt. Es ist jedoch auch mög lich, unter erhöhtem oder vermindertem Druck zu arbeiten.

Zur Durchführung des erfindungsgemäßen Verfahrens (h) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (h) jeweils nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Cheno podium, Urtica, Senecio, Amaranthus, Portulaca, Xanthium, Convolvulus, Ipomoea, Polygonum,

Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Verbindungen der Formel (I) eignen sich insbesondere zur selektiven Bekämpfung von dikotylen Unkräutern im Vorauflauf- und im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlor benzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser. Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N′-dimethyl-harnstoff

(METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage, ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); 2-[[[[(4,6-Dimethoxypyrimidin-2-yl)-amino]-carbonyl]-amino]-sulfonyl]-methyl]-benzoesäure- methylester (BENSULFURON); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 2-Chlor-N-{[(4-methoxy-6-methyl-1,3,5-triazin-2-yl)-amino]-carbonyl}-benzolsulfonamid (CHLORSULFURON); N,N-Dimethyl-N′-(3-chlor-4-methylphenyl)-harnstoff (CHLORTOLURON); 2-[4-(2,4-Dichlorphenoxy)-phenoxy]-propionsäure, deren Methyl-oder deren Ethylester (DICLOFOP); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl- oder deren Ethylester (FENOXAPROP); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N′-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-{[[((4-Methoxy-6-methyl-1,3,5-triazin-2-yl)-amino)-carbonyl]-amino]-sulfonyl}-benzoesäure oder deren Methylester (METSULFURON); S-Ethyl-N,N-hexamethylen-thiolcarbamat (MOLINATE); 4-(Di-n-propylamino)-3,5-dinitrobenzolsulfonamid (ORYZALIN); N-(1-Ethylpropyl)-3,4-dimethyl-2,6-dinitroanilin (PENDIMETHALIN); 0-(6-Chlor-3-phenylpyridazin-4-yl)-S-octyl-thiocarbonat (PYRIDATE); 2,4-Bis-[N-ethylamino]-6-methylthio-1,3,5-triazin (SIMETRYNE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); N,N-Diisopropyl-S-(2,3,3-trichlorallyl)-thiolcarbamat (TRIALLATE) und 3,5,6-Trichlor-2-pyridyloxyessigsäure (TRICLOPYR). Einige Mischungen zeigen überraschenderweise auch synergistische Wirkung. Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

## Herstellungsbeispiele

### Beispiel 1

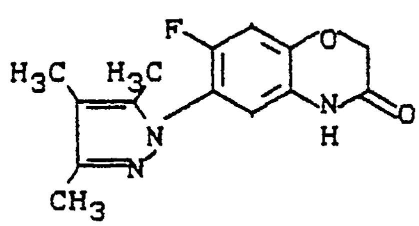

[Verfahren (a)]

Eine Mischung aus 4,0 g (0,02 Mol) 7-Fluor-6-hydrazino-3,4-dihydro-3-oxo-2H-1,4-benzoxazin, 2,3 g (0.02 Mol) 3-Methyl-2,4-pentandion (90 %ig), 20 ml Ethanol und 20 ml Wasser wird 15 Stunden unter

Rückfluß zum Sieden erhitzt. Danach wird das Lösungsmittel im Wasserstrahlvakuum abdestilliert und der Rückstand durch Chromatographie an Kieselgel (Eluens: Cyclohexan/Essigsäureethylester, 1/3) gereinigt.

Man erhält 1,1 g (20 % der Theorie) 7-Fluor-6-trimethylpyrazolyl-3,4-dihydro-3-oxo-2H-1,4-benzoxazin vom Schmelzpunkt 181 °C.

Beispiel 2

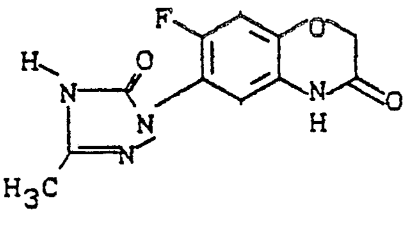

[Verfahren (b)]

Eine Mischung aus 2,36 g (0,012 Mol) 7-Fluor-6-hydrazino-3,4-dihydro-3-oxo-2H-1,4-benzoxazin, 2,86 g (0,018 Mol) N-Ethoxycarbonyl-O-ethyl-acetamidester und 25 ml Toluol wird 18 Stunden unter Rückfluß zum Sieden erhitzt. Anschließend wird das Lösungsmittel im Wasserstrahlvakuum abdestilliert und der Rückstand durch Chromatographie an Kieselgel (Eluens: Cyclohexan/Essigsäureethylester, 1/3) gereinigt.

Man erhält 0,78 g (25 % der Theorie) 7-Fluor-6-(3-methyl-5-oxo-4,5-dihydro-1H-1,2,4-triazol-1-yl)-3,4-dyhydro-3-oxo-2H-1,4-benzoxazin (Schmelzpunkt >300 °C).

$^1$H-NMR (80 Mhz, $CDCl_3$/DMSO, δ): 10,75 (bs, 1H); 7,01 (d, 1H, J = 6,6 Hz); 6,89 (d, 1H, J = 9,0 Hz); 4,57 (s, 2H); 3,32 (bs, 1H); 2,18 (s, 3H).

Beispiel 3

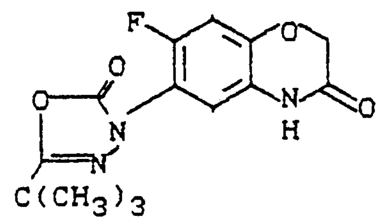

[Verfahren (c)]

Zu einer Lösung von 1,41 g (0,005 Mol) 7-Fluor-6-pivaloylhydrazino-3,4-dihydro-3-oxo-2H-1,4-benzoxazin in 50 ml Toluol werden 0,6 g (0,5 ml, 0,007 Mol) Phosgen eindosiert und dann wird unter Rühren eine Lösung von 1,52 g (0,015 Mol) Triethylamin in 2 ml Toluol tropfenweise dazu gegeben. Das Reaktionsgemisch wird dann eine Stunde bei 100 °C gerührt. Anschließend wird Stickstoff eingeleitet und im Wasserstrahlvakuum eingeengt. Der Rückstand wird durch Chromatograpie an Kieselgel (Eluens: Cyclohexan/Essigsäureethylester, 1/3) gereinigt.

Man erhält 0,55 g (36 % der Theorie) 7-Fluor-6-(2-tert-butyl-5-oxo-4,5-dihydro-1H-1,3,4-oxadiazol-3-yl)-3,4-dihydro-3-oxo-2H-1,4-benzoxazin vom Schmelzpunkt 176 °C.

$^1$H-NMR (80 Mhz, $CDCl_3$, δ): 9,68 (bs, 1H); 7,05 (d, 1H, J = 6,6 Hz); 6,85 (d, 1H, J = 9,0 Hz); 4,65 (s, 2H); 1,36 (s, 9h).

Beispiel 4

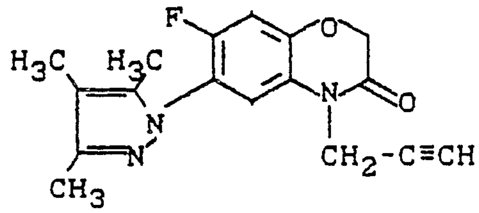

[Verfahren (e)]

0,12 g (0,004 Mol) Natriumhydrid (80 %ig in Paraffinöl) und 0,46 g (0,0038 Mol) Propargylbromid (98 %ig) werden nacheinander zu einer Lösung von 1,0 g (0,0036 Mol) 7-Fluor-6-trimethylpyrazolyl-3,4-dihydro-3-oxo-2H-1,4-benzoxazin - Herstellung vgl. Beispiel 1 - in 15 ml Dimethylsulfoxid gegeben. Das Reaktionsgemisch wird 3 Stunden bei 20 °C gerührt, dann in 50 ml Wasser gegossen und gerührt, bis der anfangs ölige Niederschlag kristallisiert. Das kristalline Produkt wird durch Absaugen isoliert.

Man erhält 0,9 g (79 % der Theorie) 7-Fluor-6-trimethylpyrazolyl-4-propargyl-3,4-dihydro-3-oxo-2H-1,4-benzoxazin vom Schmelzpunkt 189 °C.

Beispiel 5

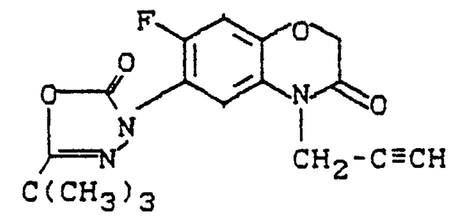

[Verfahren (e)]

Eine Mischung aus 0,46 g (0,0015 Mol) 7-Fluor-6-(2-tert-butyl-5-oxo-4,5-dihydro-1H-1,3,4-oxadiazol-3-yl)-3,4-dihydro-3-oxo-2H-1,4-benzoxazin - Herstellung vgl. Beispiel 3 - 0,30 g (0,003 Mol) Kaliumcarbonat und 8 ml Acetonitril wird eine Stunde unter Rückfluß zum Sieden erhitzt. Nach Abkühlen auf 25 °C wird eine 80 %ige Lösung von 0,35 g (0,003 Mol) Propargylbromid in Toluol dazu gegeben und das Reaktionsgemisch wird noch 4 Stunden unter Rückfluß zum Sieden erhitzt. Nach Einengen wird mit Methylenchlorid extrahiert, die Extraktionslösung mit gesättigter Natriumhydrogencarbonat-Lösung und mit Wasser gewaschen, mit Natriumsulfat getrocknet und filtriert. Vom Filtrat wird das Lösungsmittel im Wasser strahlvakuum sorgfältig abdestilliert, wobei das gewünschte Produkt als kristalliner Rückstand verbleibt.

Man erhält 0,35 g (68 % der Theorie) 7-Fluor-4-propargyl-6-(2-tert-butyl-5-oxo-4,5-dihydro-1H-1,3,4-oxadiazol-3-yl)-3,4-dihydro-3-oxo-2H-1,4-benzoxazin vom Schmelzpunkt 161 °C.

$^1$H-NMR (80 Mhz, $CDCl_3$, δ): 7,31 (d, 1H, J = 6,6 Hz); 6,90 (d, 1H, J = 9,0 Hz); 4,79 (d, 2H, J = 2,0 Hz), 4,68 (s, 2H); 2,32 (t, 1H, J = 2,0 Hz); 1,38 (s, 9H).

Analog zu den Beispielen 1 bis 5 sowie nach der allgemeinen Beschreibung der erfindungsgemäßen Herstellungsverfahren können die in der nachstehenden Tabelle 5 aufgeführten Verbindungen der Formel (I) erhalten werden.

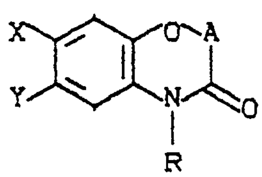

(I)

Tabelle 5: Herstellungsbeispiele für die Verbindungen der Formel (I)

| Bsp.-Nr. | A | R | X | Y | physikalische Daten |
|---|---|---|---|---|---|
| 6 | $CH_2$ | $-CH_2C{\equiv}CH$ | F | 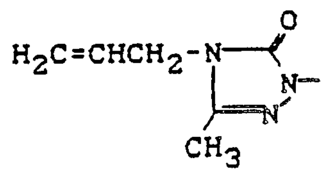 | |
| 7 | $CH_2$ | $-CH_2C{\equiv}CH$ | F | 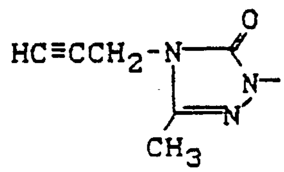 | |
| 8 | $CH_2$ | H | H | 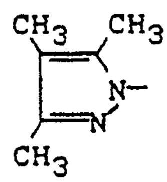 | |
| 9 | $CH_2$ | $-CH_2C{\equiv}CH$ | H | 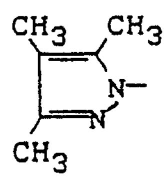 | |
| 10 | $CH_2$ | H | H | 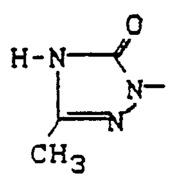 | |
| 11 | $CH_2$ | $-CH_2CH{=}CH_2$ | F | 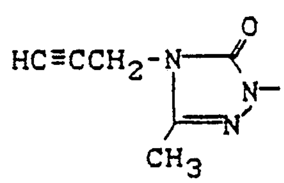 | |

Tabelle 5 - Fortsetzung

| Bsp.-Nr. | A | R | X | Y | physikalische Daten |
|---|---|---|---|---|---|
| 12 | $CH_2$ | $CH_3$ | F | 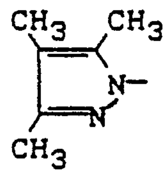 | |
| 13 | $CH_2$ | $C_2H_5$ | H | 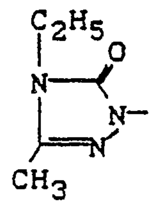 | |
| 14 | $CH_2$ | H | H | 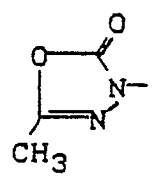 | |
| 15 | $CH_2$ | $-CH_2C \equiv CH$ | F | 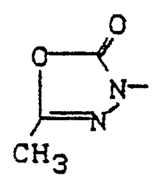 | Fp. 151 °C |
| 16 | $CH_2$ | $C_4H_9$ | H | 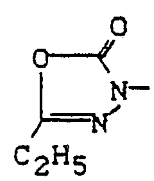 | |
| 17 | $CH_2$ | H | F | 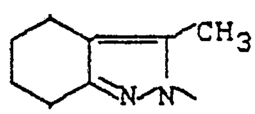 | Fp. 81 °C |
| 18 | $CH_2$ | H | F | 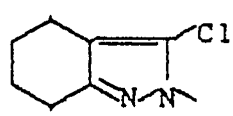 | |

Tabelle 5 - Fortsetzung

| Bsp.-Nr. | A | R | X | Y | physikalische Daten |
|---|---|---|---|---|---|
| 19 | $CH_2$ | $CH_3$ | F | 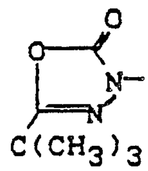 | Fp. 158-164° C |
| 20 | $CH_2$ | $CH_3$ | F | 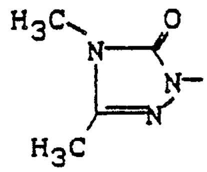 | Fp. 191-195° C |
| 21 | $CH_2$ | $-CH_2C{\equiv}CH$ | F | 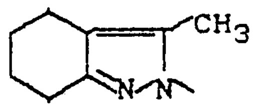 | Fp. 169-175° C |
| 22 | $CH_2$ | $-CH_2CH{=}CH_2$ | F | 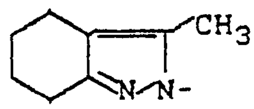 | Fp. 150 °C |
| 23 | $CH_2$ | $-CH_2C{\equiv}CH$ | F | 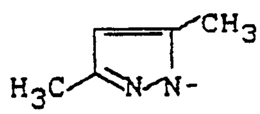 | Fp. 163 °C |
| 24 | $CH_2$ | $-CH_2C{\equiv}CH$ | F | 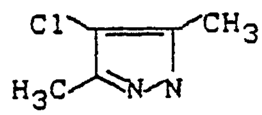 | Fp. 178 °C |
| 25 | $CH_2$ | H | F | 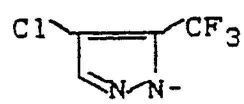 | Fp. 194 °C |
| 26 | $CH_2$ | H | F | 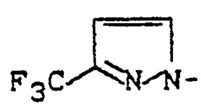 | Fp. 180 °C |
| 27 | $CH_2$ | H | F | 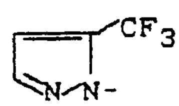 | Fp. 214 °C |

Tabelle 5 - Fortsetzung

| Bsp.-Nr. | A | R | X | Y | physikalische Daten |
|---|---|---|---|---|---|
| 28 | $CH_2$ | $-CH_2C{\equiv}CH$ | F | 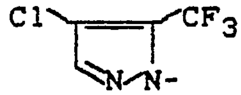 | Fp. 129 °C |
| 29 | $CH_2$ | $-CH_2C{\equiv}CH$ | F | 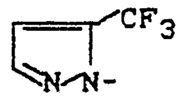 | Fp. 135 °C |
| 30 | $CH_2$ | H | H | 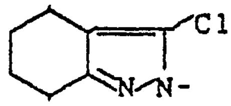 | Fp. 182 °C |
| 31 | $CH_2$ | $-CH_2C{\equiv}CH$ | H | 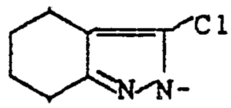 | Fp. 153 °C |
| 32 | $CH_2$ | $-CH_2CH_2OC_2H_5$ | H | 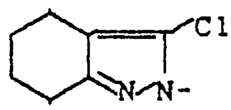 | (Öl) |
| 33 | $CH_2$ | $-CH_2COOC_2H_5$ | H | 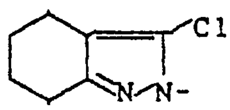 | Fp. 97 °C |
| 34 | $CH_2$ | $-CH(CH_3)-COOC_2H_5$ | H | 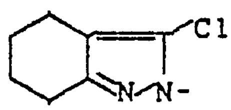 | (Öl) |
| 35 | $CH_2$ | $-CH_2CH{=}CH_2$ | H | 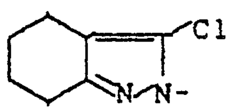 | Fp. 114 °C |
| 36 | $CH_2$ | $-CH_2-$ 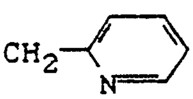 | H | 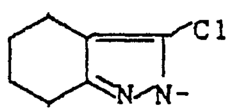 | (Öl) |
| 37 | $CH_2$ | H | F | 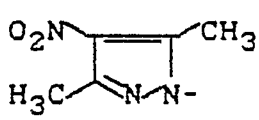 | |

Tabelle 5 - Fortsetzung

| Bsp.-Nr. | A | R | X | Y | physikalische Daten |
|---|---|---|---|---|---|
| 38 | $CH_2$ | H | F | 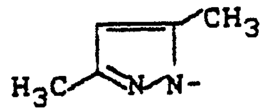 | Fp. 183° C |
| 39 | $CH_2$ | $-CH_2C\equiv CH$ | F | 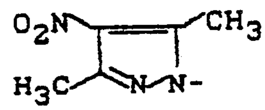 | |
| 40 | $CH_2$ | H | F | 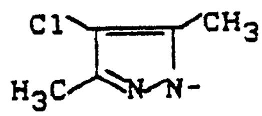 | Fp. 236° C |
| 41 | $CH_2$ | H | H | 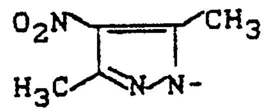 | Fp. 300° C |
| 42 | $CH_2$ | $-CH_2C\equiv CH$ | H | 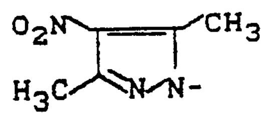 | |
| 43 | $CH_2$ | H | H | 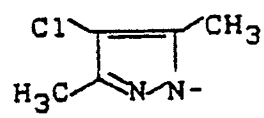 | |
| 44 | $CH_2$ | $-CH_2C\equiv CH$ | H | 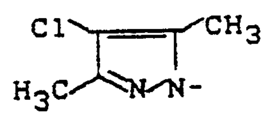 | |
| 45 | $CH_2$ | $CH_3$ | F | 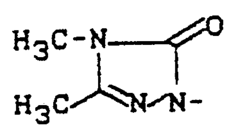 | Fp. 195 °C |
| 46 | $CH_2$ | H | F | 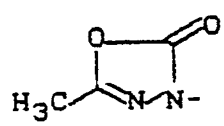 | Fp. 178 °C |
| 47 | $CH_2$ | $-CH_2CN$ | F | 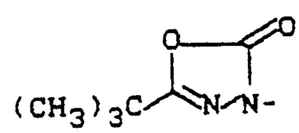 | Fp. 171 °C |

Tabelle 5 - Fortsetzung

| Bsp.-Nr. | A | R | X | Y | physikalische Daten |
| --- | --- | --- | --- | --- | --- |
| 48 | $CH_2$ | H | F | 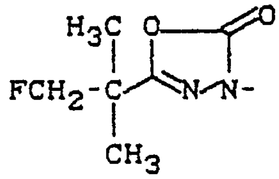 | Fp. 171 °C |
| 49 | $CH_2$ | $-CH_2C{\equiv}CH$ | F | 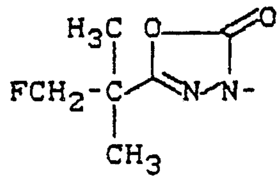 | Fp. 149 °C |
| 50 | $CH_2$ | $-CH_2C{\equiv}CH$ | F | 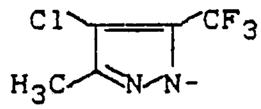 | Fp. 138 °C |
| 51 | $CH_2$ | H | F | 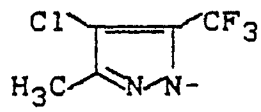 | |
| 52 | $CHCH_3$ | H | H | 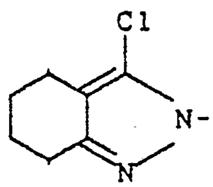 | Fp. 162° C |
| 53 | $CHCH_3$ | $-CH_2C{\equiv}CH$ | H | 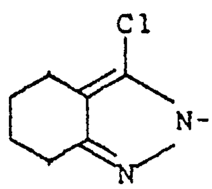 | Fp. 132° C |
| 54 | $CHCH_3$ | $-CH_2CH{=}CH_2$ | H | 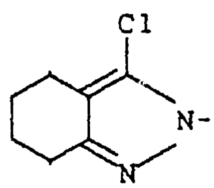 | Fp: 90° C |
| 55 | $CHCH_3$ | $-CH_2COOC_2H_5$ | | 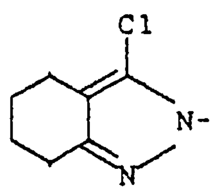 | Fp: 103° C |

Tabelle 5 - Fortsetzung

| Bsp.-Nr. | A | R | X | Y | physikalische Daten |
|---|---|---|---|---|---|
| 56 | $CH_2$ | $-CH_2CH=CH_2$ | F | 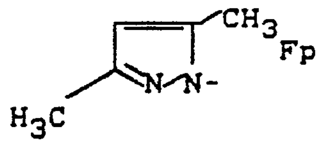 | Fp. 138 °C |
| 57 | $CH_2$ | $-CH_2CH=CH_2$ | F | 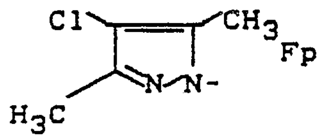 | Fp. 156 °C |
| 58 | $CH_2$ | $-CH_2C=CH_2$ | F | 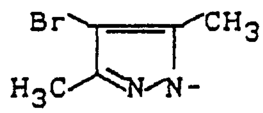 | Fp. 160 °C |
| 59 | $CH_2$ | $-CH(CH_3)COOC_2H_5$ | F | 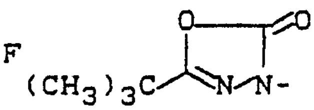 | Fp. 110-115° C |
| 60 | $CH_2$ | $-CH(CH_3)_2$ | F | 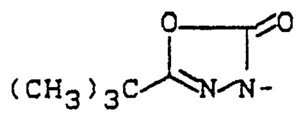 | Fp. 97-102° C |
| 61 | $CH_2$ | $-CH_2CH=CH_2$ | F | 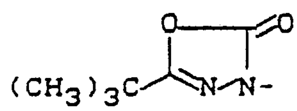 | Fp. 132-137° C |
| 62 | $CH_2$ | $-CH_2-$ 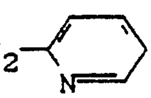 | F | 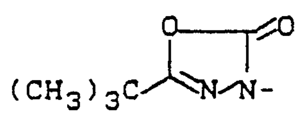 | Fp. 146° C |
| 63 | $CH_2$ | $-CH_2COOC_2H_5$ | F | 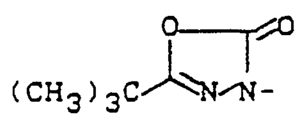 | Fp. 143° C |

Im Folgenden ist an einigen Beispielen die Herstellung von in Tabelle 5 aufgeführten Verbindungen der Formel (I) ausführlich beschrieben.

Herstellung der in Tabelle 5 als Beispiel 30 aufgeführten Verbindung:

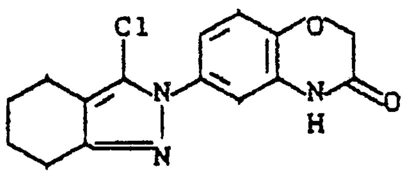

(Verfahren (d))

2,8 g (0,05 Mol) Eisenpulver werden in 8 ml Ethanol, 3 ml Wasser und 0,2 ml konz. Salzsäure 30 Minuten unter Rückfluß gerührt. Danach werden bei 65 °C bis 70 °C innerhalb von 2 Stunden in kleinen Portionen 3,9 g (0,011 Mol) 2-Nitro-4-(3-chloro-4,5,6,7-tetrahydro-2H-indazolyl)-phenoxyessigsäure zugefügt. Nach Zugabe von 10 ml Ethanol wird 2 Stunden unter Rückfluß gerührt. Das Reaktionsgemisch wird in etwa 100 ml Essigsäureethylester aufgenommen, verrührt und abgesaugt. Das Filtrat wird nach dem Ausschütteln mit Wasser und konz. Natriumhydrogencarbonatlösung über Magnesiumsulfat getrocknet und im Vakuum eingedampft.

Man erhält 1,8 g (53 % der Theorie) 2-(3,4-dihydro-3-oxo-2H-1,4-benzoxazinyl)-3-chloro-4,5,6,7-tetrahydro-2H-indazol vom Schmelzpunkt 182 °C.

Herstellung der in Tabelle 5 als Beispiel 31 aufgeführten Verbindung:

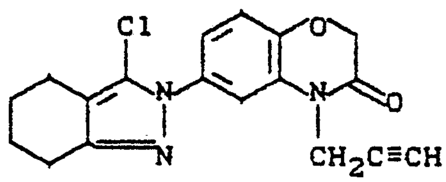

(Verfahren (e))

1,68 g (5,54 mMol) 2-(3,4-Dihydro-3-oxo-2H-1,4-benzoxazinyl)-3-chloro-4,5,6,7-tetrahydro-2H-imidazol werden in 22 ml Dimethylsulfoxid gelöst und nacheinander bei 10 °C - 15 °C mit 0,22 g (7,33 mMol) 80 %igem Natriumhydrid und 0,53 ml (7,02 mMol) Propargylbromid versetzt. Man rührt 15 Stunden bei 20 °C bis 25 °C nach. Das Reaktionsgemisch wird unter Rühren auf ca. 200 ml Wasser gegossen, der entstehende Feststoff abgesaugt, mit Wasser gewaschen und im Vakuum bei 50 °C getrocknet.

Man erhält 1,7 g (89,9 % der Theorie) 2-(3,4-Dihydro-3-oxo-4-propargyl-2H-1,4-benzoxazinyl)-3-chloro-4,5,6,7-tetrahydro-2H-indazol vom Schmelzpunkt 153 °C.

Herstellung der in Tabelle 5 als Beispiel 38 aufgeführten Verbindung:

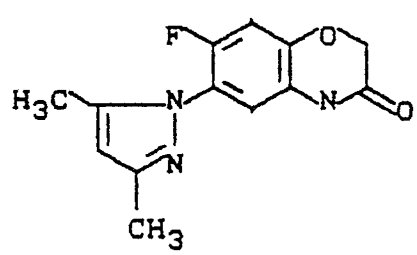

(Verfahren (a))

Eine Lösung aus 3,7 g (0,019 Mol) 7-(Fluor-6-hydrazino-3,4-dihydro-3-oxo-2H-1,4-benzoxazin, 100 ml Ethanol und 1,9 g (0,019 Mol) Pentandion werden 2 Stunden bei Raumtemperatur gerührt (Hydrazonbildung); anschließend wird 20 Stunden unter Rückfluß erhitzt. Zur Aufarbeitung wird im Vakuum das Lösungsmittel entfernt, der Rückstand in Methylenchlorid aufgenommen, zweimal mit Wasser gewaschen, über Natriumsulfat getrocknet und das Lösungsmittel im Vakuum abdestilliert. Der Rückstand wird mit Ether/Petrolether verrührt und der kristalline Niederschlag abgesaugt.

Man erhält 4,0 g (81 % der Theorie) 7-Fluor-6-(3,5-dimethylpyrazol)-3,4-dihydro-3-oxo-2H-1,4-benzoxazin vom Schmelzpunkt 168 °C.

Herstellung der in Tabelle 5 als Beispiel 23 aufgeführten Verbindung:

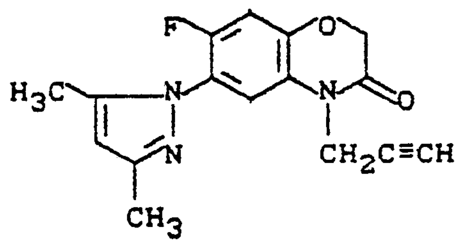

(Verfahren (e))

Eine Mischung aus 0,78 g (3 mMol) 7-Fluor-6-(3,5-dimethylpyrazolyl)-3,4-dihydro-3-oxo-2H-1,4-benzoxazin, 20 ml Acetonitril und 0,46 g (3,3 mMol) Kaliumcarbonat wird mit 0,37 g (3,2 mMol) Propargylbromid versetzt und 15 Stunden bei 60 °C gerührt. Nach Einengen wird der Rückstand in Methylenchlorid aufgenommen, mit Wasser gewaschen, über Magnesiumsulfat getrocknet und das Lösungsmittel im Vakuum entfernt.

Man erhält 0,69 g (77 % der Theorie) an 7-Fluor-6-(3,5-dimethylpyrazol)-4-propargyl-3,4-dihydro-3-oxo-2H-1,4-benzoxazin vom Schmelzpunkt 163 °C.

Herstellung der in Tabelle 5 als Beispiel 24 aufgeführten Verbindung:

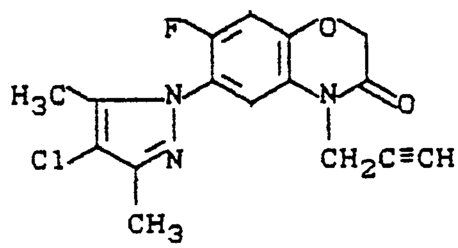

0,6 g (0,002 Mol) 7-Fluor-6-(3,5-dimethylpyrazol)-4-propargyl-3,4-dihydro-3-oxo-2H-1,4-benzoxazin in 30 ml Methylenchlorid werden mit 0,2 ml (0,0025 Mol) Sulfurylchlorid versetzt. Man läßt 20 Stunden bei 35 °C nachrühren. Anschließend wird die Reaktionsmischung mit gesättigter Natriumchloridlösung gewaschen, über Natriumsulfat getrocknet und das Lösungsmittel im Vakuum entfernt.

Man erhält 0,4 g (60 % der Theorie) an 7-Fluor-6-(3,5-dimethyl-4-chlorpyrazol)-4-propargyl-3,4-dihydro-3-oxo-2H-1,4-benzoxazin vom Schmelzpunkt 178 °C.

Ausgangsstoffe der Formel (II)

Beispiel (II-1)

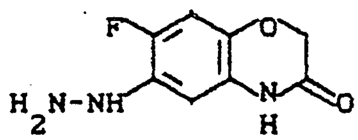

Eine Lösung von 8,8 g (0,13 Mol) Natriumnitrit in 65 ml 98 %iger Schwefelsäure wird unter Rühren zu einer Mischung aus 20 g (0,11 Mol) 6-Amino-7-fluor-3,4-dihydro-3-oxo-2H-1,4-benzoxazin und 270 ml Essigsäure gegeben und das Reaktionsgemisch wird 3 Stunden bei 15 °C bis 25 °C gerührt. Dann wird bei 10 °C eine Lösung von 80 g (0,35 Mol) Zinn-(II)-chlorid-Dihydrat in 70 ml 36 %iger Salzsäure dazu gegeben, die Mischung weitere 15 Stunden bei 20 °C gerührt und dann auf 5 °C abgekühlt. Das kristallin angefallene Produkt wird durch Filtration isoliert, in 300 ml 25 %iger Ammoniaklösung suspendiert und auf 5 °C abgekühlt. Das kristalline Produkt wird wiederum durch Filtration isoliert, an der Luft getrocknet und mit Essigsäureethylester extrahiert. Von der Extraktionslösung wird das Lösungsmittel im Wasserstrahlvakuum sorgfältig abdestilliert.

Man erhält 10,4 g (48 % der Theorie) 7-Fluor-6-hydrazino-3,4-dihydro-3-oxo-2H-1,4-benzoxazin als öligen Rückstand.

$^1$H-NMR ($D_6$-DMSO, $\delta$): 6,35 (d, 1H); 6,72 (d, 1H); 4,42 (s, 2H).

Analog Beispiel (II-1) können die in der nachstehenden Tabelle 6 aufgeführten Verbindungen der Formel (II) erhalten werden.

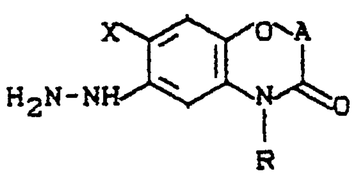

(II)

**Tabelle 6**: Beispiele für die Ausgangsstoffe der Formel (II)

| Bsp.-Nr. | A | R | X | physikalische Daten |
|---|---|---|---|---|
| II-2 | $CH_2$ | H | H | |
| II-3 | $CH_2$ | $CH_3$ | H | |
| II-4 | $CH_2$ | $CH_3$ | F | |
| II-5 | $CH_2$ | $-CH_2C\equiv CH$ | H | |
| II-6 | $CH_2$ | $-CH_2C\equiv CH$ | F | |
| II-7 | $CH_2$ | $-CH_2CH=CH_2$ | H | |
| II-8 | $CH_2$ | $-CH_2CH=CH_2$ | F | |
| II-9 | $CH_2$ | $-CH_2CN$ | H | |
| II-10 | $CH_2$ | $-CH_2CN$ | F | |
| II-11 | $CH_2$ | $-CH_2COOC_2H_5$ | F | |
| II-12 | $CH_2$ | $-CH_2CH_2OC_2H_5$ | F | |
| II-13 | $CH_2$ | $-\underset{\vert \atop CH_3}{C}HCOOC_2H_5$ | F | |
| II-14 | $CH_2$ | $-CH_2-$ 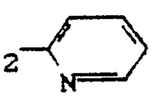 | F | |

Ausgangsstoffe der Formel (V)

Beispiel (V-1)

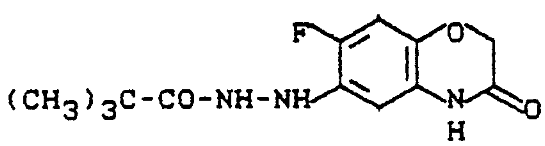

5,35 g (0,053 Mol) Pivaloylchlorid werden tropfenweise zu einer auf 0 °C gekühlten Mischung aus 6,30 g (0,032 Mol) 7-Fluor-6-hydrazino-3,4-dihydro-3-oxo-2H-1,4-benzoxazin, 6,3 g (0,053 Mol) Triethylamin und 150 ml Methylenchlorid unter Rühren gegeben und die Reaktionsmischung wird 10 Stunden bei 20 °C gerührt. Anschließend wird eingeengt und der Rückstand mit Essigsäureethylester/Wasser geschüttelt. Die organische Phase wird mit Natriumsulfat getrocknet und filtriert. Das Filtrat wird eingeengt und der Rückstand durch Chromatographie an Kieselgel (Eluens: Cyclohexan/Essigester, 1/3) gereinigt.

Man erhält 4,75 g (53 % der Theorie) 7-Fluor-6-pivaloylhydrazino-3,4-dihydro-3-oxo-2H-1,4-benzoxazin

vom Schmelzpunkt 195 °C.
$^1$H-NMR (80 Mhz, DMSO-$D_6$, δ): 10,40 (s, 1H); 9,30 (d, 1H, J =2,0 Hz); 6,64 (d, 1H, J =9,0); 6,52 (d, 1H, J = 7,4 Hz); 6,36 (d, 1H, J =2,= Hz); 4,43 (s, 2H); 1,25 (s, 9H).

Analog Beispiel (V-1) können die in der nachstehenden Tabelle 7 aufgeführten Verbindungen der Formel (V) erhalten werden.

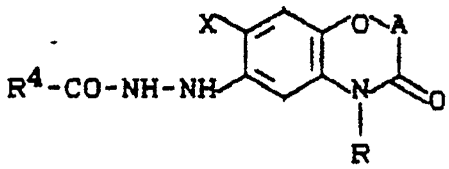

(V)

Tabelle 7: Beispiele für die Verbindungen der Formel (V)

| Bsp.-Nr. | A | R | $R^4$ | X | physikalische Daten |
|---|---|---|---|---|---|
| V-2 | $CH_2$ | H | $C(CH_3)_3$ | H | |
| V-3 | $CH_2$ | H | $CH_3$ | H | |
| V-4 | $CH_2$ | H | $CH_3$ | F | Fp. 230 °C |
| V-5 | $CH_2$ | $-CH_3$ | $CH_3$ | F | |
| V-6 | $CH_2$ | $-CH_2CN$ | $CH_3$ | F | |
| V-7 | $CH_2$ | $-CH_2CH=CH_2$ | $CH_3$ | H | |
| V-8 | $CH_2$ | $-CH_2CH=CH_2$ | $CH_3$ | F | |
| V-9 | $CH_2$ | $-CH_2C \equiv CH$ | $CH_3$ | H | |
| V-10 | $CH_2$ | $-CH_2C \equiv CH$ | $CH_3$ | F | |
| V-11 | $CH_2$ | $-CH_2C \equiv CH$ | $C_2H_5$ | F | |
| V-12 | $CH_2$ | $-CH_2C \equiv CH$ | $C_3H_7$ | F | |
| V-13 | $CH_2$ | $-CH_2C \equiv CH$ | $C_4H_9$ | F | |
| V-14 | $CH_2$ | $-CH_2CN$ | $C(CH_3)_3$ | F | |
| V-15 | $CH_2$ | H | $C(CH_3)_2CH_2F$ | F | Fp. 179 °C |
| V-16 | $CH_2$ | H | $C(CH_3)(CH_2F)_2$ | F | |

Ausgangsstoffe der Formel (VI)

Beispiel (VI-1/VI-2)

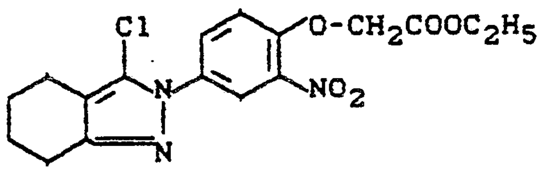

(VI-1)

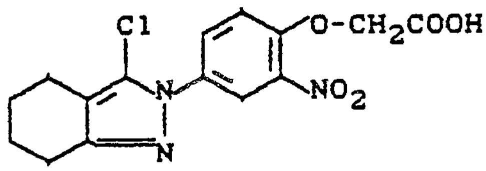

(VI-2)

10 g (0,03 Mol) 4-(3-Chlor-4,5,6,7-tetrahydro-2H-imidazolyl)-phenoxyessigsäureethylester werden in 33 ml konz. Schwefelsäure gelöst und auf -20 °C abgekühlt. Es werden 1,9 ml 100 %ige Salpetersäure unter Rühren bie -20 °C bis -15 °C langsam zugetropft. Dann wird 3 Stunden bei dieser Temperatur nachgerührt. Anschließend wird der Ansatz unter Rühren auf Eiswasser gegossen. Der Niederschlag wird abgesaugt und in Essigsäureethylester gelöst. Die organische Lösung wird mit gesättigter Natriumhydrogencarbonatlösung ausgeschüttelt, über Magnesiumsulfat getrocknet und im Vakuum eingedampft.

Man erhält 4,6 g (41 % der Theorie) 2-Nitro-4-(3-chlor-4,5,6,7-tetrahydro-2H-indazolyl)-phenoxyessigsäureethylester als öliges Produkt.

NMR (Dimethylsulfoxid) $\delta$ = 7,47 ppm (1H, Dublett)

Die beim Aufarbeiten erhaltene Natriumhydrogencarbonat lösung wird mit Wasser verdünnt und unter Rühren mit Salzsäure sauer gestellt. Der ausgefallene Niederschlag wird abgesaugt, mit Wasser gewaschen und im Vakuum bei 50 °C getrocknet.

Man erhält 4,1 g (39 % der Theorie) 2-Nitro-4-(3-chlor-4,5,6,7-tetrahydro-2H-indazolyl)-phenoxyessigsäure vom Schmelzpunkt 167 °C - 172 °C.

Ausgangsstoffe der Formel (XII)

Beispiel (XII-1)

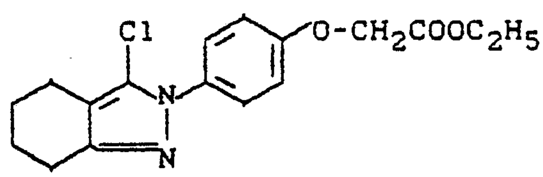

9,9 g (0,04 Mol) 2-(4-Hydroxyphenyl)-3-chlor-4,5,6,7-tetrahydro-2H-indazol werden in 100 ml Acetonitril gelöst, nacheinander mit 6,1 g (0,044 Mol) Kaliumcarbonat sowie 7,4 ml (0,066 Mol) Bromessigsäureethylester versetzt und 6 Stunden unter Rückfluß gerührt. Das Reaktionsgemisch wird im Vakuum eingedampft, in Methylenchlorid gelöst, dreimal mit Wasser ausgeschüttelt, über Magnesiumsulfat getrocknet und erneut im Vakuum eingedampft.

Man erhält 13,4 g (100 % der Theorie) 4-(3-Chlor-4,5,6,7-tetrahydro-2H-indazolyl)-phenoxyessigsäuremethylester als öliges Produkt.

NMR ($CDCl_3$) $\delta$ = 4,64 ppm (2H, Singulett)

Ausgangsstoffe der Formel (XIV)

Beispiel (XIV-1)

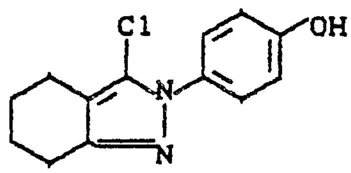

49,0 g (0,178 Mol) 2-(4-Methoxyphenyl)-3-chlor-4,5,6,7-tetrahydro-2H-indazol werden in 450 ml 48 %iger Bromwasserstoffsäure 10 Stunden unter leichtem Rückfluß gerührt. Das Reaktionsgemisch wird im Vakuum eingedampft, der so erhaltene Feststoff mit Essigsäureethylester heiß verrührt, abgesaugt und zur Beseitigung des anhaltenden Bromwasserstoffs 3 Stunden mit gesättigter Natriumhydrogencarbonatlösung verrührt. Danach wird abgesaugt, mit Wasser gewaschen und im Vakuum bei 50 °C getrocknet.

Man erhält 35,7 g (77 % der Theorie) 2-(4-Hydroxyphenyl)-3-chlor-4,5,6,7-tetrahydro-2H-indazol vom Schmelzpunkt 217 °C - 223 °C.

Ausgangsstoffe der Formel (XVII).

Beispiel (XVII-1)

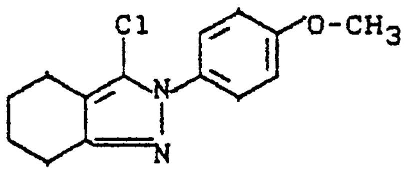

1. Stufe

5,8 g (0,25 Mol) Natrium werden in 220 ml Ethanol gelöst. Durch die Lösung wird Argon geleitet. Es werden 43,6 g (0,25 Mol) 4-Methoxy-phenylhydrazin-hydrochlorid zugesetzt und ca. 40 Minuten unter Argon bei 40 °C gerührt. Danach werden 42,6 g (0,25 Mol) 2-Oxo-cyclohexancarbonsäureethylester zugegeben und 5 Stunden unter Rückfluß gerührt. Das entstandene Natriumchlorid wird abgesaugt und das erhaltene Filtrat im Wasserstrahlvakuum eingedampft. Der Rückstand wird zwecks Reinigung mit Petrolether verrührt und anschließend im Vakuum bei 50 °C getrocknet.

Man erhält 57,1 g (93,5 % der Theorie) 2-(4-Methoxyphenyl)-3-hydroxy-4,5,6,7-tetrahydro-2H-indazol vom Schmelzpunkt 159 °C.

2. Stufe

42,7 g (0,175 Mol) 2-(4-Methoxyphenyl)-3-hydroxy-4,5,6,7-tetrahydro-2H-indazol werden in 75 ml Phosphoroxychlorid 19 Stunden unter Rückfluß gerührt. Das überschüssige Phosphoroxychlorid wird im Vakuum abgedampft, der ölige Rückstand in Methylenchlorid gelöst und mit Wasser, 2 %iger Salzsäure, gesättigter Natriumhydrogencarbonatlösung und gesättigter Natriumchloridlösung gewaschen. Die Lösung wird über Magnesiumsulfat getrocknet und anschließend im Vakuum eingedampft.

Man erhält 25,7 g ( 55,9 % der Theorie) 2-(4-Methoxyphenyl)-3-chlor-4,5,6,7-tetrahydro-2H-indazol als öliges Produkt.

NMR ($CDCl_3$) = 3,85 ppm (3H, Singulett)

Anwendungsbeispiele

In den folgenden Anwendungsbeispielen wird die nachstehend aufgeführte Verbindung als Vergleichssubstanz herangezogen:

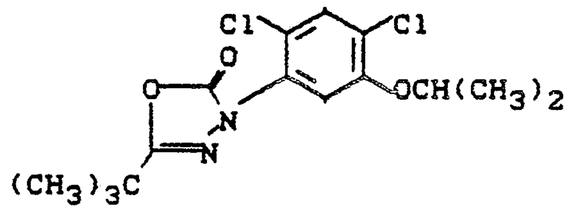

(A)

5-tert-Butyl-3-(2,4-dichlor-5-isopropoxy-phenyl)-1,3,4-oxadiazol-2-on (Oxadiazone/ Ronstar) - bekannt aus US-P 3 835 862.

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit gegenüber der Vergleichssubstanz (A) zeigen in diesem Test z. B. die Verbindungen gemäß Herstellungsbeispiel 3, 4, 5, 22, 24, 30, 31, 35 und 49.

Beispiel B

Post-ermergence-Test

Lösungsmittel: 5 Gewichtsteil Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

Eine deutliche Überlegenheit in der Wirksamkeit gegenüber der Vergleichssubstanz (A) zeigt in diesem Test z.B. die Verbindungen gemäß der Herstellungsbeispiele 3, 4, 5, 22, 31, 32, 33, 34, 35, 47 und 49.

## Ansprüche

1. Substituierte Benzoxazinone der Formel (I)

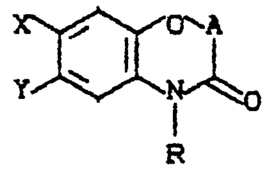

(I)

in welcher

A

für Methylen ($-CH_2-$), Ethyliden ($-\overset{\overset{CH_3}{|}}{C}H-$) oder

Isopropyliden ($-\underset{\underset{CH_3}{|}}{\overset{\overset{CH_3}{|}}{C}}-$) steht,

R für Wasserstoff oder für einen gegebenenfalls substituierten Rest aus der Reihe Alkyl, Cycloalkyl, Cycloalkylalkyl, Alkenyl, Alkinyl, Aralkyl und Heteroarylalkyl steht,

X für Wasserstoff oder Halogen steht und

Y für die nachstehenden heterocyclischen Gruppierungen steht

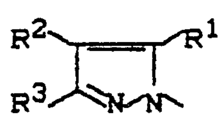

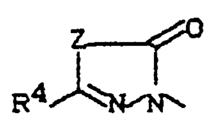

worin

$R^1$ für Wasserstoff, Halogen oder gegebenenfalls durch Halogen substituiertes Alkyl steht,

$R^2$ für Wasserstoff, Cyano, Nitro, Halogen oder gegebenenfalls durch Halogen substituiertes Alkyl steht,

$R^3$ für Wasserstoff oder gegebenenfalls durch Halogen substituiertes Alkyl steht, oder zusammen mit $R^2$ für Alkandiyl steht,

$R^4$ für Wasserstoff oder für jeweils gegebenenfalls durch Halogen substituiertes Alkyl, Cycloalkyl, Alkenyl oder Alkinyl steht und

Z für Sauerstoff oder die Gruppierung $-N-R^5$ steht, worin

$R^5$ für Wasserstoff oder für jeweils gegebenenfalls durch Halogen substituiertes Alkyl, Alkenyl oder Alkinyl steht, oder zusammen mit $R^4$ für Alkandiyl steht.

2. Substituierte Benzoxaninone der Formel (I) gemäß Anspruch 1, in welcher

A

für Methylen ($-CH_2-$), Ethyliden 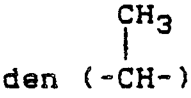 oder Isopropyliden 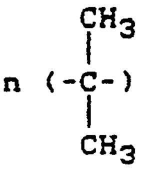 steht,

R für Wasserstoff, für gegebenenfalls durch Fluor, Chlor, Brom, Iod, Cyano, Carboxy, Carbamoyl, $C_1$-$C_4$-Alkoxy, $C_1$-$C_4$-Alkoxy-$C_1$-$C_2$-alkoxy, $C_1$-$C_4$-Alkoxy-carbonyl, $C_1$-$C_4$-Alkylamino-carbonyl, $C_1$-$C_4$-Alkoxy-carbonyloxy, $C_1$-$C_4$-Alkoxy-carbonylamino, Aminocarbonyloxy, Aminocarbonylamino und/oder $C_1$-$C_4$-Alkylaminocarbonylamino substituiertes $C_1$-$C_8$-Alkyl, für gegebenenfalls durch Fluor, Chlor, Brom, Iod, Cyano, Carboxy, Carbamoyl und/oder $C_1$-$C_4$-Alkoxy-carbonyl substituiertes $C_3$-$C_8$-Alkenyl, für gegebenenfalls durch Fluor, Chlor, Brom, Iod, Cyano und/oder $C_1$-$C_4$-Alkoxy-carbonyl substituiertes $C_3$-$C_8$-Alkinyl, für gegebenenfalls durch Fluor, Chlor, Brom, Iod und/oder $C_1$-$C_4$-Alkyl substituiertes $C_3$-$C_6$-Cycloalkyl, für gegebenenfalls durch Fluor, Chlor, Brom, Iod und/oder $C_1$-$C_4$-Alkyl substituiertes $C_3$-$C_6$-Cycloalkyl-$C_1$-$C_4$-alkyl, für gegebenenfalls durch Fluor, Chlor, Brom,
Cyano, $C_1$-$C_4$-Alkyl, $C_1$-$C_4$-Alkoxy und/oder $C_1$-$C_4$-Alkoxy-carbonyl substituiertes Benzyl oder für gegebenenfalls durch Fluor, Chlor, Brom, Iod, Cyano, $C_1$-$C_4$-Alkyl und/oder $C_1$-$C_4$-Alkoxy substituiertes Heteroarylalkyl aus der Reihe Furyl-$C_1$-$C_4$-alkyl, Thienyl-$C_1$-$C_4$-alkyl, Oxazolyl-$C_1$-$C_4$-alkyl, Oxadiazolyl-$C_1$-$C_4$-alkyl, Thiazolyl-$C_1$-$C_4$-alkyl, Thiadiazolyl-$C_1$-$C_4$-alkyl, Pyridinyl-$C_1$-$C_4$-alkyl und Pyrimidinyl-$C_1$-$C_4$-alkyl steht,
X für Wasserstoff, Fluor oder Chlor steht und
Y für die nachstehenden heterocyclischen Gruppierungen steht

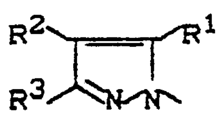

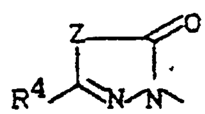

worin
$R^1$ für Wasserstoff, Fluor, Chlor, Brom oder für gegebenenfalls durch Fluor und/oder Chlor substituiertes $C_1$-$C_4$-Alkyl steht,
$R^2$ für Wasserstoff, Cyano, Nitro, Fluor, Chlor, Brom, Iod oder für gegebenenfalls durch Fluor und/oder Chlor substituiertes $C_1$-$C_4$-Alkyl steht,
$R^3$ für Wasserstoff oder gegebenenfalls durch Fluor und/oder Chlor substituiertes $C_1$-$C_4$-Alkyl steht oder zusammen mit $R^2$ für $C_3$- oder $C_4$-Alkandiyl steht,
$R^4$ für Wasserstoff oder für jeweils gegebenenfalls durch Fluor, Chlor und/oder Brom substituiertes $C_1$-$C_4$-Alkyl, $C_3$-$C_6$-Cycloalkyl, $C_2$-$C_4$-Alkenyl oder $C_2$-$C_4$-Alkinyl steht, und
Z für Sauerstoff oder für die Gruppierung $-N-R^5$ steht, worin
$R^5$ für Wasserstoff oder für jeweils gegebenenfalls durch Fluor und/oder Chlor substituiertes $C_1$-$C_4$-Alkyl, $C_3$-$C_4$-Alkenyl oder $C_3$-$C_4$-Alkinyl steht, oder zusammen mit $R^4$ für $C_3$-oder $C_4$-Alkandiyl steht.

3. Verfahren zur Herstellung von substituierten Benzoxazinonen der Formel (I)

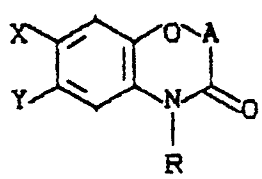

(I)

in welcher
A

für Methylen ($-CH_2-$), Ethyliden ($-\overset{CH_3}{\overset{|}{CH}}-$) oder

Isopropyliden ($-\overset{CH_3}{\overset{|}{\underset{CH_3}{\underset{|}{C}}}}-$) steht,

steht,
R für Wasserstoff oder für einen gegebenenfalls substituierten Rest aus der Reihe Alkyl, Cycloalkyl, Cycloalkylalkyl, Alkenyl, Alkinyl, Aralkyl und Heteroarylalkyl steht,
X für Wasserstoff oder Halogen steht und
Y für die nachstehenden heterocyclischen Gruppierungen steht

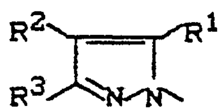

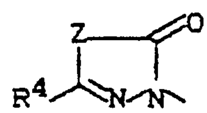

worin
$R^1$ für Wasserstoff, Halogen oder gegebenenfalls durch Halogen substituiertes Alkyl steht,
$R^2$ für Wasserstoff, Cyano, Nitro, Halogen oder gegebenenfalls durch Halogen substituiertes Alkyl steht,
$R^3$ für Wasserstoff oder gegebenenfalls durch Halogen substituiertes Alkyl steht, oder zusammen mit $R^2$ für Alkandiyl steht,
$R^4$ für Wasserstoff oder für jeweils gegebenenfalls durch Halogen substituiertes Alkyl, Cycloalkyl, Alkenyl oder Alkinyl steht und
Z für Sauerstoff oder die Gruppierung $-N-R^5$ steht, worin
$R^5$ für Wasserstoff oder für jeweils gegebenenfalls durch Halogen substituiertes Alkyl, Alkenyl oder Alkinyl steht, oder zusammen mit $R^4$ für Alkandiyl steht,
dadurch gekennzeichnet, daß man

(a) Hydrazino-benzoxazinone der allgemeinen Formel (II)

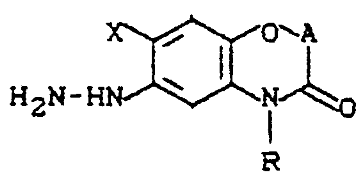

(II)

in welcher
A, R und X die oben angegebenen Bedeutungen haben,
mit 1,3-Dicarbonylverbindungen der allgemeinen Formel (III)

$$R^1-CO-\underset{R^2}{\underset{|}{CH}}-CO-R^3 \qquad (III)$$

in welcher
$R^1$, $R^2$ und $R^3$ die oben angegebenen Bedeutungen haben,
bzw. mit Dimethyl- oder Diethyl-Acetalen bzw. -Ketalen von Verbindungen der Formel (III) gegebenenfalls in Gegenwart von Verdünnungsmitteln umsetzt, oder daß man

(b) die bei Verfahren (a) beschriebenen Hydrazino-benzoxazinone der allgemeinen Formel (II)

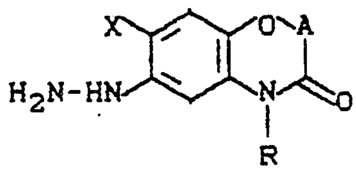

(II)

in welcher
A, R und X die oben angegebenen Bedeutungen haben,
mit N-Alkoxycarbonylcarbonimidsäureestern der allgemeinen Formel (IV)

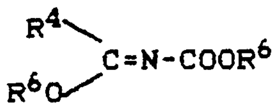

(IV)

in welcher
$R^4$ die oben angegebene Bedeutung hat und
$R^6$ für Niederalkyl steht,
gegebenenfalls in Gegenwart eines Verdünnungsmittels umsetzt, oder daß man

(c) Acylhydrazino-benzoxazinone der allgemeinen Formel (V)

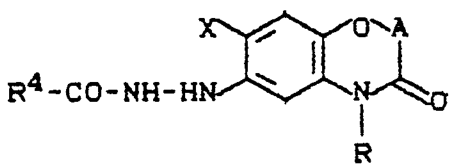

(V)

in welcher
A, R, $R^4$ und X die oben angegebenen Bedeutungen haben,
mit Phosgen gegebenenfalls in Gegenwart eines Säureakzeptors und gegebenenfalls in Gegenwart eines Verdünnungsmittels umsetzt, oder daß man

(d) Verbindungen der allgemeinen Formel (VI)

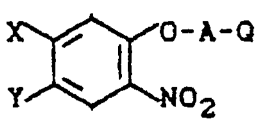

(VI)

in welcher
A, X und Y die oben angegebenen Bedeutungen haben und
Q für Cyano, Carboxy oder $C_1$-$C_4$-Alkoxycarbonyl steht,
mit einem Reduktionsmittel, gegebenenfalls in Gegenwart eines Katalysators und in Gegenwart eines Verdünnungsmittels umsetzt, oder daß man

(e) Benzoxazinone der allgemeinen Formel (Ia)

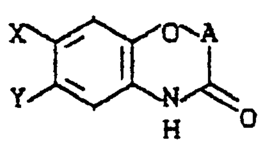

(Ia)

in welcher
A, X und Y die oben angegebenen Bedeutungen haben,
mit Alkylierungsmitteln der allgemeinen Formel (VII)

R - $X^1$ (VII)

in welcher
R die oben angegebene Bedeutung hat und
X' für Halogen steht,
gegebenenfalls in Gegenwart eines Säureakzeptors und gegebenenfalls in Gegenwart eines Verdünnungsmittels umsetzt, oder daß man

(f) Benzoxazinone der allgemeinen Formel (Ib)

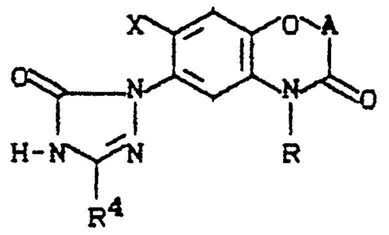

(Ib)

in welcher
A, R, $R^4$ und X die oben angegebenen Bedeutungen haben,
mit Alkylierungsmitteln der allgemeinen Formel (VIII)

$R^5 - X^2$ (VIII)

in welcher
$R^5$ für jeweils gegebenenfalls durch Halogen substituiertes Alkyl, Alkenyl oder Alkinyl steht und
$X^2$ für Halogen steht,
gegebenenfalls in Gegenwart eines Säureakzeptors und gegebenenfalls in Gegenwart eines Verdünnungsmittels umsetzt, oder daß man

(g) Benzoxazinone der allgemeinen Formel (IX)

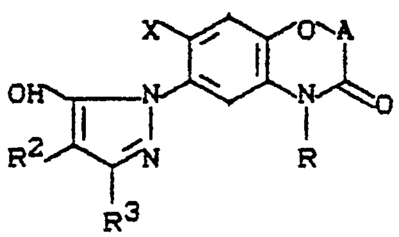

(IX)

in welcher
A, R, $R^2$, $R^3$ und X die oben angegebenen Bedeutungen haben,
mit einem Halogenierungsmittel, gegebenenfalls in Gegenwart eines Katalysators und gegebenenfalls in Gegenwart eines Verdünnungsmittels umsetzt, oder daß man

(h) Benzoxazinone der allgemeinen Formel (Ic)

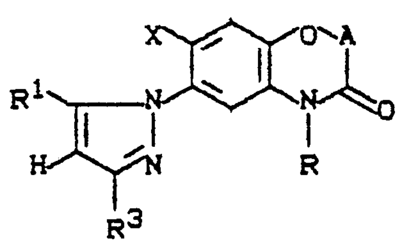

(Ic)

in welcher
A, R, $R^1$, $R^3$ und X die oben angegebenen Bedeutungen haben,
mit einem Halogenierungsmittel oder Nitrierungsmittel, gegebenenfalls in Gegenwart eines Katalysators und gegebenenfalls in Gegenwart eines Verdünnungsmittel, umsetzt.

4. Herbizide Mittel, gekennzeichnet durch einen Gehalt an mindestens einem substituierten Benzoxazinon der Formel (I) gemäß den Ansprüchen 1 bis 3.

5. Verfahren zur Bekämpfung von Unkräutern, dadurch gekennzeichnet, daß man substituierte Benzoxazinone der Formel (I) gemäß den Ansprüchen 1 bis 3 auf die Unkräuter und/oder ihren Lebensraum einwirken läßt.

6. Verwendung von substituierten Benzoxazinonen der Formel (I) gemäß den Ansprüchen 1 bis 3 zur Bekämpfung von Unkräutern.

7. Verfahren zur Herstellung von herbiziden Mitteln, dadurch gekennzeichnet, daß man substituierte Benzoxazinone der Formel (I) gemäß den Ansprüchen 1 bis 3 mit Streckmitteln und/oder oberflächenaktiven Substanzen vermischt.

8. Hydrazino-benzoxazinone der Formel (IIa)

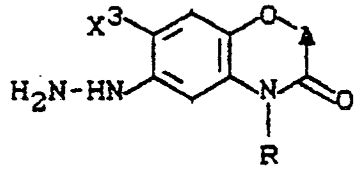

(IIa)

in welcher

$X^3$ für Halogen steht,

A

für Methylen ($-CH_2-$), Ethyliden ($-CH(CH_3)-$) oder

Isopropyliden ($-C(CH_3)_2-$)

steht und

R für Wasserstoff oder für einen gegebenenfalls substituierten Rest aus der Reihe Alkyl, Cycloalkyl, Cycloalkylalkyl, Alkenyl, Alkinyl, Aralkyl und Heteroarylalkyl steht.

9. Acylhydrazino-benzoxazinone der Formel (V)

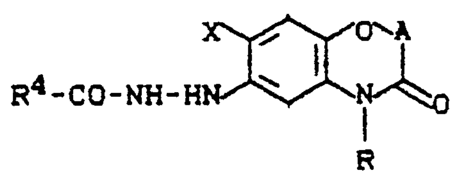

(V)

in welcher

A

**für Methylen ($-CH_2-$), Ethyliden** 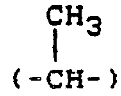 **oder**

**Isopropyliden** 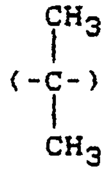 **steht und**

steht und

R für Wasserstoff oder für einen gegebenenfalls substituierten Rest aus der Reihe Alkyl, Cycloalkyl, Cycloalkylalkyl, Alkenyl, Alkinyl, Aralkyl und Heteroarylalkyl steht,

$R^4$ für Wasserstoff oder für jeweils gegebenenfalls durch Halogen substituiertes Alkyl, Cycloalkyl, Alkenyl oder Alkinyl steht und

X für Wasserstoff oder Halogen steht.

10. Verbindungen der Formel (VI)

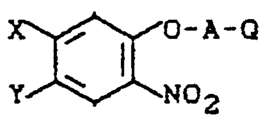

(VI)

in welcher

A

**für Methylen ($-CH_2-$), Ethyliden** 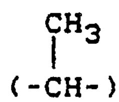 **oder**

**Isopropyliden** 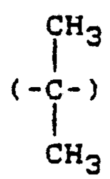 **steht,**

steht,

X für Wasserstoff oder Halogen steht,

Y für die nachstehenden heterocyclischen Gruppierungen steht

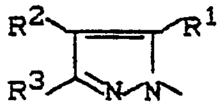 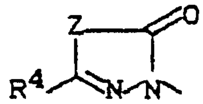

worin

$R^1$ für Wasserstoff, Halogen oder gegebenenfalls durch Halogen substituiertes Alkyl steht,

$R^2$ für Wasserstoff, Cyano, Nitro, Halogen oder gegebenenfalls durch Halogen substituiertes Alkyl steht,

$R^3$ für Wasserstoff oder gegebenenfalls durch Halogen substituiertes Alkyl steht, oder zusammen mit $R^2$ für Alkandiyl steht,

$R^4$ für Wasserstoff oder für jeweils gegebenenfalls durch Halogen substituiertes Alkyl, Cycloalkyl, Alkenyl oder Alkinyl steht und

Z für Sauerstoff oder die Gruppierung $-N-R^5$ steht, worin

$R^5$ für Wasserstoff oder für jeweils gegebenenfalls durch Halogen substituiertes Alkyl, Alkenyl oder Alkinyl steht, oder zusammen mit $R^4$ für Alkandiyl steht und

Q für Cyano, Carboxy oder $C_1$-$C_4$-Alkoxycarbonyl steht.

11. Benzoxazinone der Formel (IX)

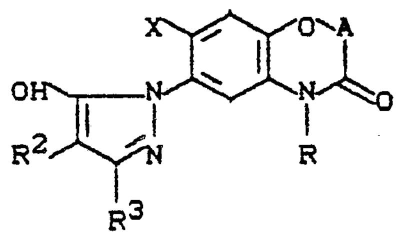

(IX)

in welcher

A

für Methylen ($-CH_2-$), Ethyliden ($-\overset{\overset{\large CH_3}{|}}{C}H-$) oder

Isopropyliden ($-\underset{\underset{\large CH_3}{|}}{\overset{\overset{\large CH_3}{|}}{C}}-$) steht und

steht und

R für Wasserstoff oder für einen gegebenenfalls substituierten Rest aus der Reihe Alkyl, Cycloalkyl, Cycloalkylalkyl, Alkenyl, Alkinyl, Aralkyl und Heteroarylalkyl steht,

$R^2$ für Wasserstoff, Cyano, Nitro, Halogen oder gegebenenfalls durch Halogen substituiertes Alkyl steht,

$R^3$ für Wasserstoff oder gegebenenfalls durch Halogen substituiertes Alkyl steht, oder zusammen mit $R^2$ für Alkandiyl steht,

X für Wasserstoff oder Halogen steht.

12. Verbindungen der Formel (XII)

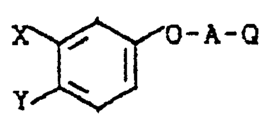

(XII)

in welcher

A

für Methylen ($-CH_2-$), Ethyliden ($-\overset{\overset{\large CH_3}{|}}{C}H-$) oder

Isopropyliden ($-\underset{\underset{\large CH_3}{|}}{\overset{\overset{\large CH_3}{|}}{C}}-$) steht,

steht,
Q für Cyano, Carboxy oder $C_1$-$C_4$-Alkoxycarbonyl steht,
X für Wasserstoff oder Halogen steht und
Y für die nachstehenden heterocyclischen Gruppierungen steht

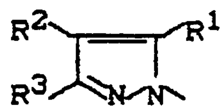 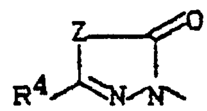

worin
$R^1$ für Wasserstoff, Halogen oder gegebenenfalls durch Halogen substituiertes Alkyl steht,
$R^2$ für Wasserstoff, Cyano, Nitro, Halogen oder gegebenenfalls durch Halogen substituiertes Alkyl steht,
$R^3$ für Wasserstoff oder gegebenenfalls durch Halogen substituiertes Alkyl steht, oder zusammen mit $R^2$ für Alkandiyl steht,
$R^4$ für Wasserstoff oder für jeweils gegebenenfalls durch Halogen substituiertes Alkyl, Cycloalkyl, Alkenyl oder Alkinyl steht und
Z für Sauerstoff oder die Gruppierung -N-$R^5$ steht, worin
$R^5$ für Wasserstoff oder für jeweils gegebenenfalls durch Halogen substituiertes Alkyl, Alkenyl oder Alkinyl steht, oder zusammen mit $R^4$ für Alkandiyl steht.

13. Verfahren zur Herstellung von Hydrazino-benzoxazinonen der Formel (IIa)

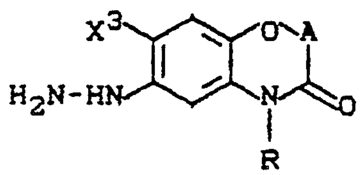

(IIa)

in welcher
$X^3$ für Halogen steht,
A

$CH_3$

A für Methylen ($-CH_2-$), Ethyliden ($-CH-$)

oder

$CH_3$

Isopropyliden ($-C-$) steht und

$CH_3$

steht und
R für Wasserstoff oder für einen gegebenenfalls substituierten Rest aus der Reihe Alkyl, Cycloalkyl, Cycloalkylalkyl, Alkenyl, Alkinyl, Aralkyl und Heteroarylalkyl steht, dadurch gekennzeichnet, daß man Amino-benzoxazinone der Formel (X)

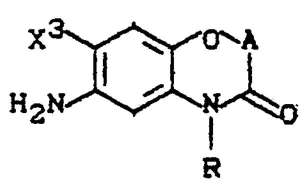

(X)

in welcher
A, R und $X^3$ die oben angegebene Bedeutung haben,
mit Nitriten in Gegenwart von Säuren und gegebenenfalls in Gegenwart von Verdünnungsmitteln und anschließend mit Reduktionsmitteln in Gegenwart von Säuren umsetzt.

14. Verfahren zur Herstellung von Acylhydrazino-benzoxazinonen der Formel (V)

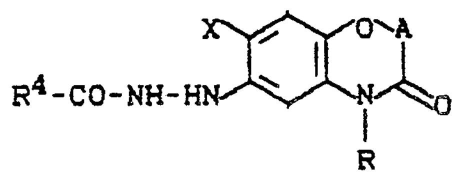

(V)

in welcher
A

für Methylen ($-CH_2-$), Ethyliden ($-CH(CH_3)-$) oder

Isopropyliden ($-C(CH_3)_2-$) steht,

steht,
R für Wasserstoff oder für einen gegebenenfalls substituierten Rest aus der Reihe Alkyl, Cycloalkyl, Cycloalkylalkyl, Alkenyl, Alkinyl, Aralkyl und Heteroarylalkyl steht,
$R^4$ für Wasserstoff oder für jeweils gegebenenfalls durch Halogen substituiertes Alkyl, Cycloalkyl, Alkenyl oder Alkinyl steht und
X für Wasserstoff oder Halogen steht,
dadurch gekennzeichnet, daß man Hydrazino-benzoxazinone der Formel (II)

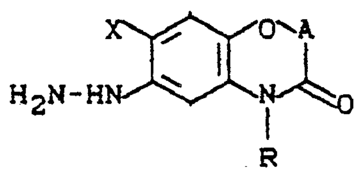

(II)

in welcher
A, R und X die oben angegebenen Bedeutungen haben,
mit Acylierungsmitteln der allgemeinen Formel (XI)
$R^4 - CO - X^4$ (XI)
in welcher
$R^4$ die oben angegebene Bedeutung hat und
$X^4$ für Halogen steht,
gegebenenfalls in Gegenwart von Säureakzeptoren und gegebenenfalls in Gegenwart von Verdünnungsmitteln umsetzt.

15. Verfahren zur Herstellung von Verbindungen der Formel (VI)

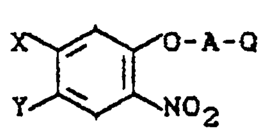

(VI)

in welcher
A

CH3
für Methylen ($-CH_2-$), Ethyliden (-CH-) oder

CH3
Isopropyliden (-C-) steht,
CH3

steht,
X für Wasserstoff oder Halogen steht,
Y für die nachstehenden heterocyclischen Gruppierungen steht

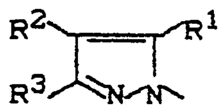

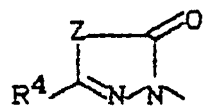

worin
$R^1$ für Wasserstoff, Halogen oder gegebenenfalls durch Halogen substituiertes Alkyl steht,
$R^2$ für Wasserstoff, Cyano, Nitro, Halogen oder gegebenenfalls durch Halogen substituiertes Alkyl steht,
$R^3$ für Wasserstoff oder gegebenenfalls durch Halogen substituiertes Alkyl steht, oder zusammen mit $R^2$ für Alkandiyl steht,
$R^4$ für Wasserstoff oder für jeweils gegebenenfalls durch Halogen substituiertes Alkyl, Cycloalkyl, Alkenyl oder Alkinyl steht und
Z für Sauerstoff oder die Gruppierung $-N-R^5$ steht, worin
$R^5$ für Wasserstoff oder für jeweils gegebenenfalls durch Halogen substituiertes Alkyl, Alkenyl oder Alkinyl steht, oder zusammen mit $R^4$ für Alkandiyl steht, und
Q für Cyano, Carboxy oder $C_1$-$C_4$-Alkoxycarbonyl steht,
dadurch gekennzeichnet, daß man Verbindungen der Formel (XII)

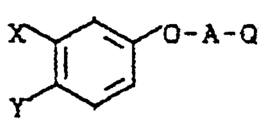

(XII)

in welcher
A, Q, X und Y die oben angegebenen Bedeutungen haben,
mit Nitrierungsmitteln, gegebenenfalls in Gegenwart von Schwefelsäure, umsetzt.

16. Verfahren zur Herstellung von Benzoxazinonen der Formel (IX)

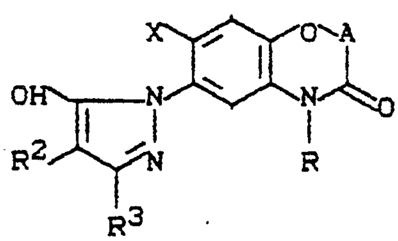

(IX)

in welcher

A

$CH_3$
|
für Methylen ($-CH_2-$), Ethyliden (-CH-) oder

$CH_3$
|
Isopropyliden (-C-) steht,
|
$CH_3$

steht,
R für Wasserstoff oder für einen gegebenenfalls substituierten Rest aus der Reihe Alkyl, Cycloalkyl, Cycloalkylalkyl, Alkenyl, Alkinyl, Aralkyl und Heteroarylalkyl steht,
$R^2$ für Wasserstoff, Cyano, Nitro, Halogen oder für gegebenenfalls durch Halogen substituiertes Alkyl steht,
$R^3$ für Wasserstoff oder gegebenenfalls durch Halogen substituiertes Alkyl steht oder zusammen mit $R^2$ für Alkandiyl steht und
X für Wasserstoff oder Halogen steht,
dadurch gekennzeichnet, daß man Hydrazinobenzoxazinone der Formel (II)

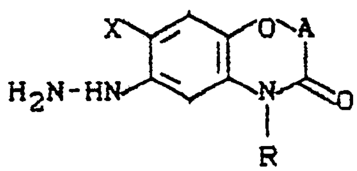
(II)

in welcher
A, R und X die oben angegebenen Bedeutungen haben,
mit β-Ketoestern der allgemeinen Formel (XX)

$R^3-CO-CH-COOR^8$ (XX)
|
$R^2$

in welcher
$R^2$ und $R^3$ die oben angegebenen Bedeutungen haben und
$R^8$ für Alkyl steht,
gegebenenfalls in Gegenwart von Verdünnungsmitteln, umsetzt.

17. Verfahren zur Herstllung von Verbindungen der Formel (XII)

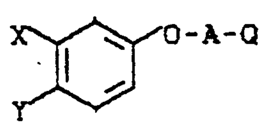
(XII)

in welcher
A

$CH_3$
für Methylen ($-CH_2-$), Ethyliden ($-CH-$) oder

$CH_3$
Isopropyliden ($-C-$) steht und
$CH_3$

steht und
Q für Cyano, Carboxy oder $C_1$-$C_4$-Alkoxycarbonyl steht,
X für Wasserstoff oder Halogen steht und
Y für die nachstehenden heterocyclischen Gruppierungen steht

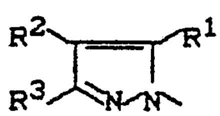  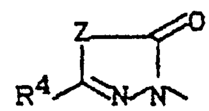

worin
$R^1$ für Wasserstoff, Halogen oder gegebenenfalls durch Halogen substituiertes Alkyl steht,
$R^2$ für Wasserstoff, Cyano, Nitro, Halogen oder gegebenenfalls durch Halogen substituiertes Alkyl steht,
$R^3$ für Wasserstoff oder gegebenenfalls durch Halogen substituiertes Alkyl steht, oder zusammen mit $R^2$ für Alkandiyl steht,
$R^4$ für Wasserstoff oder für jeweils gegebenenfalls durch Halogen substituiertes Alkyl, Cycloalkyl, Alkenyl oder Alkinyl steht und
Z für Sauerstoff oder die Gruppierung $-N-R^5$ steht, worin
$R^5$ für Wasserstoff oder für jeweils gegebenenfalls durch Halogen substituiertes Alkyl, Alkenyl oder Alkinyl steht, oder zusammen mit $R^4$ für Alkandiyl steht,
dadurch gekennzeichnet, daß man Phenolderivate der Formel (XIV)

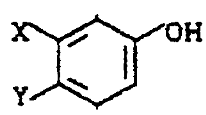 (XIV)

in welcher
X und Y die oben angegebenen Bedeutungen haben,
mit Alkylierungsmitteln der Formel (XV)
$X^5 - A - Q$ (XV)
in welcher
A und Q die oben angegebenen Bedeutungen haben und
$X^5$ für Halogen steht,
in Gegenwart eines Säureakzeptors und in Gegenwart eines Verdünnungsmittels umsetzt.